(19) **Europäisches Patentamt / European Patent Office / Office européen des brevets**

(11) **EP 2 381 902 B1**

(12) # EUROPEAN PATENT SPECIFICATION

(45) Date of publication and mention
of the grant of the patent:
**21.09.2016 Bulletin 2016/38**

(51) Int Cl.:
***A61F 5/01*** (2006.01)

(21) Application number: **09789515.5**

(22) Date of filing: **13.03.2009**

(86) International application number:
**PCT/US2009/037171**

(87) International publication number:
**WO 2010/062407 (03.06.2010 Gazette 2010/22)**

(54) **WEIGHT-BEARING LOWER EXTREMITY BRACE**

BELASTBARE BEINSCHIENE

ATTELLE POUR ARTICULATION PORTANTE D'EXTRÉMITÉ INFÉRIEURE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **26.11.2008 US 324185**

(43) Date of publication of application:
**02.11.2011 Bulletin 2011/44**

(73) Proprietor: **Toad Corporation
Incline Village, NV 89451 (US)**

(72) Inventors:
• **FRANKE, Hans, G.
Incline Village
NV 89451 (US)**

• **SALINGER, David
Olympic Valley
CA 96146 (US)**
• **DODD, Jeff
Truckee
CA 96161 (US)**

(74) Representative: **Lawrence, John
Barker Brettell LLP
100 Hagley Road
Edgbaston
Birmingham B16 8QQ (GB)**

(56) References cited:
**WO-A-97/49359        WO-A-2006/053283
FR-A- 2 432 306       US-A1- 2003 216 675
US-A1- 2005 054 962   US-A1- 2008 269 656
US-B1- 6 228 044      US-B1- 6 997 891**

**Description**

The present invention relates, according to some embodiments, to methods, devices, and systems for ambulation of a subject having an impaired lower extremity. Subjects unable to support their own weight due to a lower extremity impairment may resort to crutches or wheel chairs to move about. However, crutches and wheel chairs maybe undesirable because of the limitations they impose on a subject's ability to use their hands. In addition, crutches and/or wheel chairs may incompletely restore mobility, and/or may be untenable in an older person due to decreased upper body strength and/or poor balance in the case of crutches.

US Patent Application Publication US 2005/0054962 discloses a suspension walker having a hard outer boot shell with upright brace members attached on either side and a soft boot receivable thereon. US Patent US 6,997,891 discloses a leg support system for transferring weight support from the ankle region of a leg to the upper portion of the calf just below the knee. French Patent Application FR 2 432 306 discloses a support structure for immobilization of a leg. US Patent Application Publication US 2003/0216675 , on which the preamble of appended claim 1 is based, discloses a custom-made ankle/foot orthosis for the treatment of patients having plantar ulcers. international Application WO 97/49359 discloses an orthopedic bandage for the treatment of trauma to the Achilles tendon. US patent application US 2008/0269656 discloses an orthopaedic device for treating neuropathic ulcers whilst allowing access to the wound site on the plaintiff surface of a patient's foot.

Accordingly, a need has arisen for improved ambulatory devices for subjects with impairments of the lower extremities. This and other objects can be achieved by the system and method as defined in the independent claims. Further enhancements are characterized in the dependent claims. The present disclosure relates, according to some embodiments, to methods, devices, and systems for ambulation of a subject having an impaired lower extremity. For example, a device may comprise a lower extremity brace configured to bear a subject's weight and/or transfer the load to unimpaired organs and/or tissues.

In accordance with the present invention, a system for redistributing weight away from a subject's foot comprises (a) a platform, (b) a single vertical support fixed to the platform and extending upwardly from the platform, and (c) at least one cuff (i) configured to surround and releasably grip at least a portion of a subject's leg other than the foot and (ii) adjustably mounted to the vertical support so as to be mounted at a vertical position along the at least one vertical support sufficient to suspend a subject's foot in a non-weight-bearing position above the platform during ambulation, wherein the platform, the vertical support, and the at least one cuff together are configured to bear at least the subject's full weight. A vertical support may comprise, in some embodiments, a strut positioned relative to the subject's leg on which the system is worn opposite the subject's sagittal plane or opposite the subject's coronal plane. For example, a strut may be positioned generally behind the subject's leg on which the system is worn. In some embodiments, a strut positioned generally behind the subject's leg on which the system is worn may have an S-curve profile. A vertical support may comprise, for example, a strut configured to extend along the outside of the leg on which the system is worn. In some embodiments, a vertical support may extend distal to the subject's foot (e.g., extend from about the tibial plateau to beyond the bottom of the subject's foot). In some embodiments, a cuff may comprise an anterior shell, an opposing posterior shell, and at least one tension adjustment fastener configured to releasably connect the anterior shell and the posterior shell, wherein the anterior shell and the posterior shell are configured to combine to surround the circumference of the subject's leg. A cuff may comprise at least one collar (*e.g.*, adjustably mounted to the vertical support), according to some embodiments. A collar may have, in some embodiments, opposing ends spaced apart and a tension adjustment fastener corresponding to each collar, each tension adjustment fastener configured to releasably connect the opposing ends of the corresponding collar, wherein each collar and corresponding tension adjustment fastener are together configured to surround the circumference of the subject's leg.

According to some embodiments, a cuff may further comprise at least one pad shell fixed to the at least one collar, and at least one pad fixed to the at least one pad shell. A pad shell may comprise a moldable plastic selected from the group consisting of a thermoplastic, a thermosetting plastic, and combinations thereof, in some embodiments. A pad may comprise, according to some embodiments, a material selected from the group consisting of polyurethane, polyethylene, neoprene, ethylene vinyl acetate, foam, silicone, rubber, and combinations thereof. In some embodiments, a cuff may include a second pad shell fixed to the at least one collar and a second pad fixed to the second pad shell. A cuff including two pads and pad shells may be arranged such that the first pad comprises an anterior pad, the first pad shell comprises an anterior pad shell, the second pad comprises a posterior pad, and the second pad shell comprises a posterior pad shell, according to some embodiments. A cuff may be configured to extend from about the gastrocnemius/solius muscles to about the tibial plateau, according to some embodiments. A device and/or system (*e.g.*, for redistributing weight away from a subject's foot) may comprise, in some embodiments, an outsole fixed to the platform and configured to contact the ground during ambulation. In some embodiments, an outsole may comprise a flange extending, for example, vertically along at least a portion of the back of the vertical support.

A shell (*e.g.*, anterior shell), according to some embodiments, may comprise at least one fastener guide positioned across the anterior surface of the anterior shell and configured to receive at least a portion of the at least one tension adjustment fastener. In some embodiments, a shell (*e.g.*, anterior shell, posterior shell) may comprise a moldable plastic selected from the group consisting of a thermoplastic, a thermosetting plastic, and combinations thereof. A shell (*e.g.*, anterior shell, posterior shell) may be mounted (*e.g.*, fixedly, adjustably) to the vertical support in some embodiments. A shell (*e.g.*, anterior shell, posterior shell), according to some embodiments, may include an attached pad. For example, a cuff may include one or more pads fixed to (*e.g.*, lining) at least a portion of the surface(s) proximal to the subject's skin. Each cuff and/or each shell may include a pad. Each pad may independently comprise a material selected from the group consisting of polyurethane, polyethylene, neoprene, ethylene vinyl acetate, foam, silicone, rubber, and combinations thereof. In some embodiments, a cuff may comprise one or more bladders fixed to (*e.g.*, lining) at least a portion of the surface(s) proximal to the subject's skin. Each cuff and/or each shell may include a bladder. Each bladder may be filled (*e.g.*, at least partially filled, completely filled) with a fill material. A fill material may comprise a foam. A fill material may provide, for example, a cushioned and/or custom fit.

According to some embodiments, a device and/or system (*e.g.*, for redistributing weight away from a subject's foot) may be configured to secure a subject's leg in a bent (*e.g.*, slightly bent) position. A vertical support may comprise, for example, a vertical rod (*e.g.*, extending distal to the subject's foot). A device and/or system (*e.g.*, for redistributing weight away from a subject's foot) may comprise, according to some embodiments, a second cuff, the second cuff (a) configured to surround and releasably grip at least a second portion of a subject's leg other than the foot and (b) mounted (*e.g.*, fixedly, adjustably) to the vertical support at a vertical position along the vertical support sufficient to suspend a subject's foot in a non-weight-bearing position above the platform during ambulation.

In some embodiments, a device and/or system (*e.g.*, for redistributing weight away from a subject's foot) may comprise further (d) a hinge attached to the upper end of the vertical support, (e) a second vertical support rotatably attached to the hinge, and (f) a second cuff, the second cuff (i) configured to surround and releasably grip at least a second portion of a subject's leg other than the foot and (ii) mounted (*e.g.*, fixedly, adjustably) to the second vertical support at a vertical position along the second vertical support sufficient to cooperate with the first cuff to suspend a subject's foot in a non-weight-bearing position above the platform during ambulation. A second cuff may be configured to extend from about the proximal patella to about the upper quadriceps. According to some embodiments, a device and/or system (*e.g.*, for redistributing weight away from a subject's foot) may comprise a mated shoe comprising a lift configured to vertically align the suspended foot and the other foot. In some embodiments, a strut may be configured to be contiguous with its adjacent platform.

The present invention relates, in some embodiments, to methods for facilitating ambulation of a subject having a leg with an impaired lower extremity. A method may comprise, for example, suspending at least a portion of the impaired lower extremity in a non-weight bearing position using a load redistribution system according to the present invention and redistributing the weight to one or more unimpaired regions of the leg using the load redistribution system. Redistributing the weight to one or more unimpaired regions of the leg may comprise, in some embodiments, redistributing the weight to one or more lateral surfaces (*e.g.*, unimpaired lateral surfaces) of the leg. According to some embodiments, a method may comprise adjusting the pressure applied to at least one lateral surface (*e.g.*, by substituting with a different contact surface area). A method (*e.g.*, for facilitating ambulation of a subject having a leg with an impaired lower extremity) may comprise, for example, contacting the leg with a load redistribution system to suspend at least the impaired lower extremity in a non-weight bearing position and redistributing at least the weight of the subject during ambulation to one or more unimpaired regions of the leg.

Some embodiments of the present invention may be understood by referring, in part, to the present disclosure and the accompanying drawings, wherein:

FIGURE 1A illustrates a generally isometric view of a device for ambulation of a subject having an impaired lower extremity according to a specific example embodiment of the disclosure;

FIGURE 1B illustrates a left profile view of the device for ambulation of a subject shown in FIGURE 1A according to a specific example embodiment of the disclosure;

FIGURE 1C illustrates a right profile view of the device for ambulation of a subject shown in FIGURE 1A according to a specific example embodiment of the disclosure;

FIGURE 1D illustrates a front view of the device for ambulation of a subject shown in FIGURE 1A according to a specific example embodiment of the disclosure;

FIGURE 2A illustrates a generally isometric view of a device for ambulation of a subject having an impaired lower extremity according to a specific example embodiment of the disclosure;

FIGURE 2B illustrates a generally isometric view of a device for ambulation of a subject having an impaired lower extremity according to a specific example embodiment of the disclosure;

FIGURE 3A illustrates a generally isometric view of a device for ambulation of a subject having an impaired lower extremity according to a specific example embodiment of the disclosure;

FIGURE 3B illustrates a left profile view of the device for ambulation of a subject shown in FIGURE 3A according to a specific example embodiment of the disclosure;

FIGURE 3C illustrates a right profile view of the device for ambulation of a subject shown in FIGURE 3A according to a specific example embodiment of the disclosure;

FIGURE 3D illustrates a front view of the device for ambulation of a subject shown in FIGURE 3A according to a specific example embodiment of the disclosure;

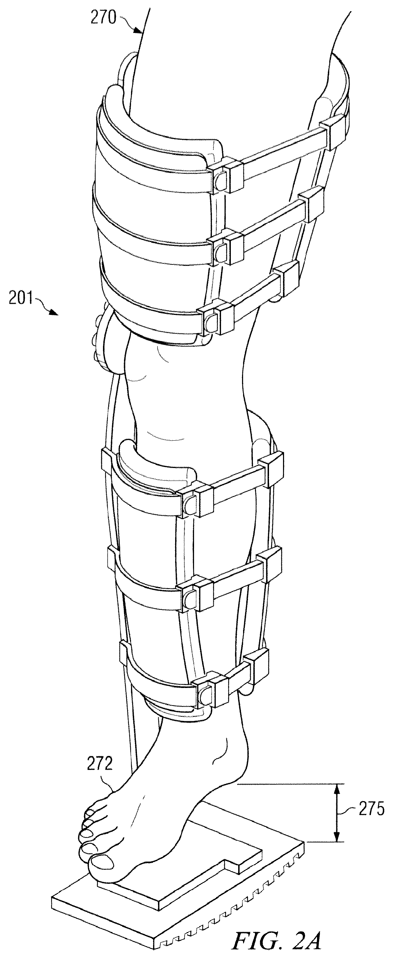

FIGURE 2A illustrates a generally isometric view of a device for ambulation of a subject having an impaired lower extremity according to a specific example embodiment of the disclosure;

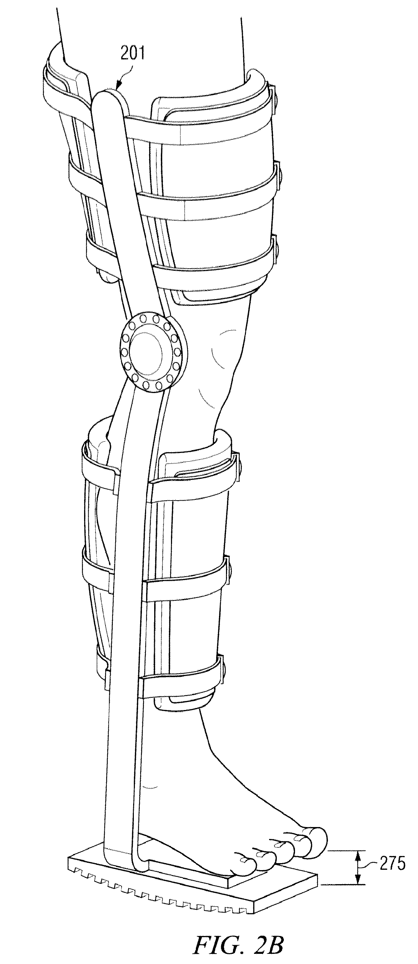

FIGURE 2B illustrates a generally isometric view of a device for ambulation of a subject having an impaired lower extremity according to a specific example embodiment of the disclosure;

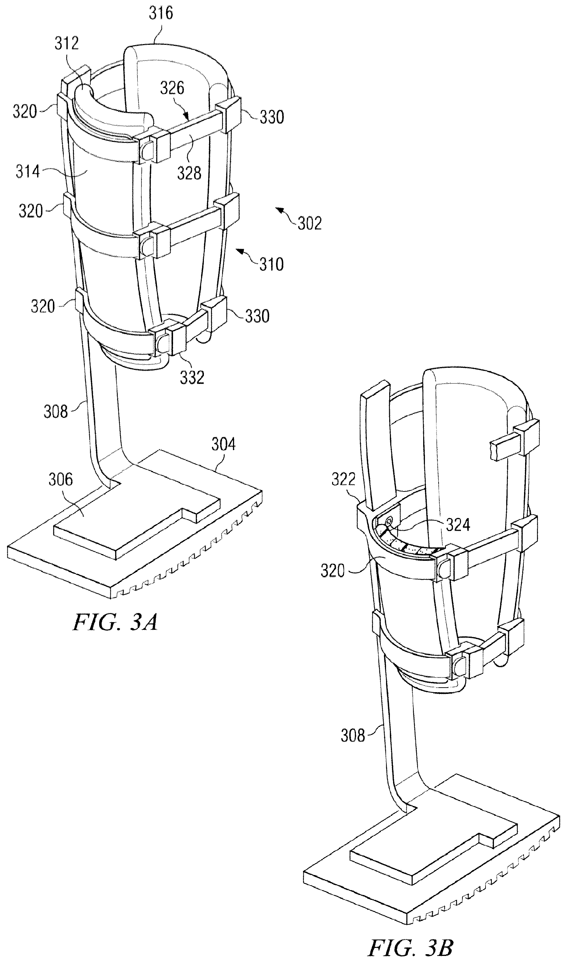

FIGURE 3A illustrates a generally isometric view of a device for ambulation of a subject having an impaired lower extremity according to a specific example embodiment of the disclosure;

FIGURE 3B illustrates a left profile view of the device for ambulation of a subject shown in FIGURE 3A according to a specific example embodiment of the disclosure;

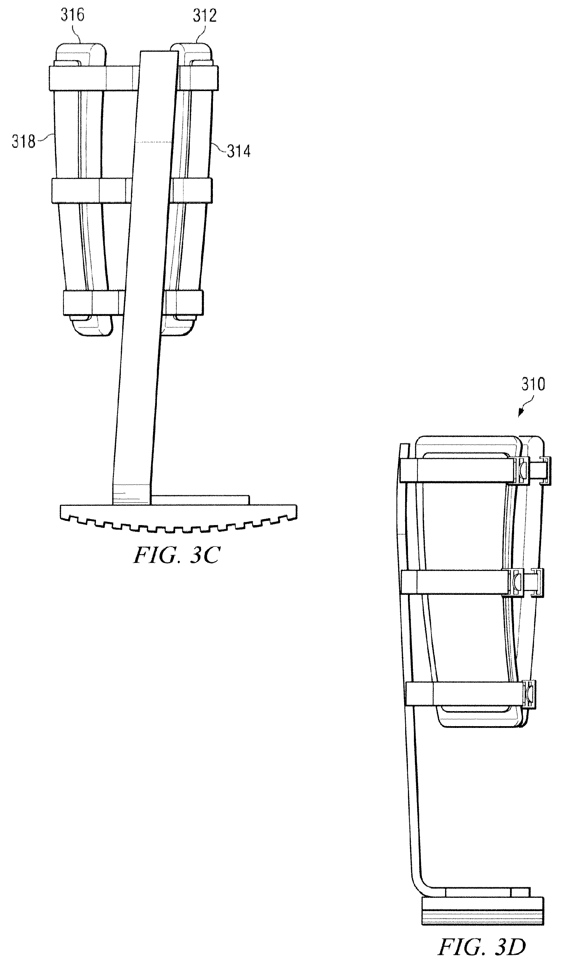

FIGURE 3C illustrates a right profile view of the device for ambulation of a subject shown in FIGURE 3A according to a specific example embodiment of the disclosure;

FIGURE 3D illustrates a front view of the device for ambulation of a subject shown in FIGURE 3A according to a specific example embodiment of the disclosure;

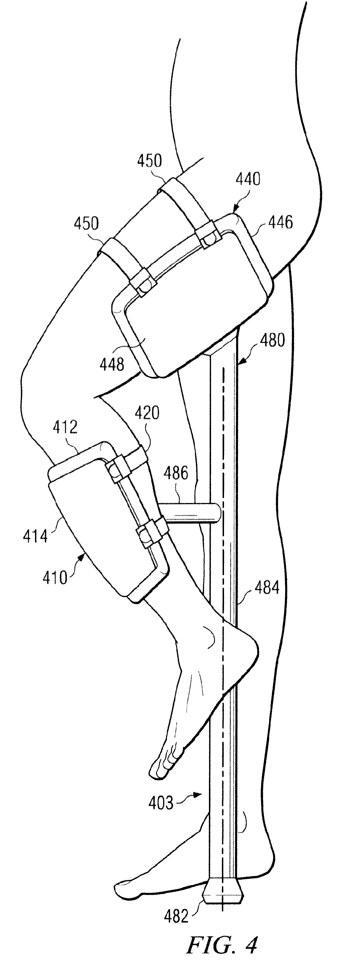

FIGURE 4 illustrates a profile view of a device for ambulation of a subject having an impaired lower extremity which is not part of the present invention as defined in the claims;

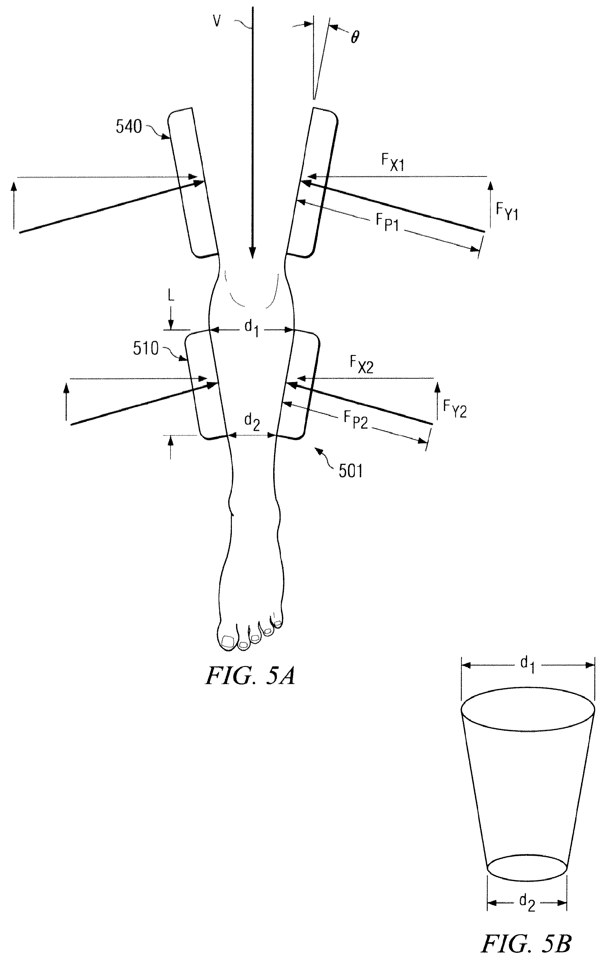

FIGURE 5A illustrates a front view of a device for ambulation of a subject having an impaired lower extremity according to a specific example embodiment of the disclosure;

FIGURE 5B illustrates a generally isometric view of a cuff according to a specific example embodiment of the disclosure;

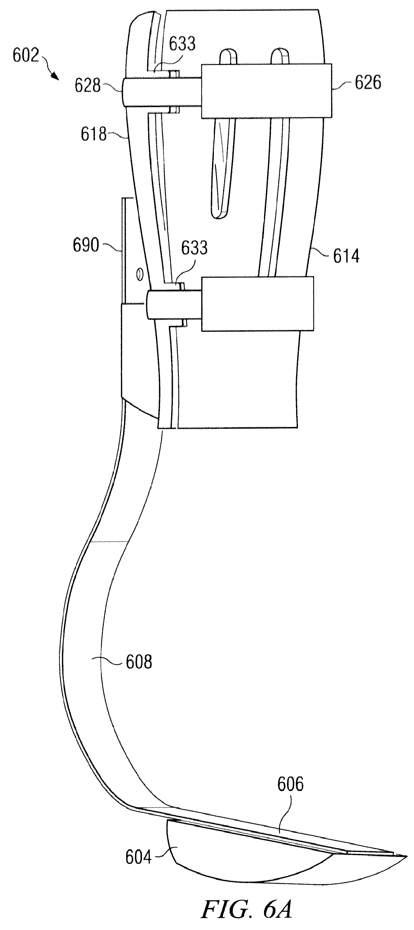

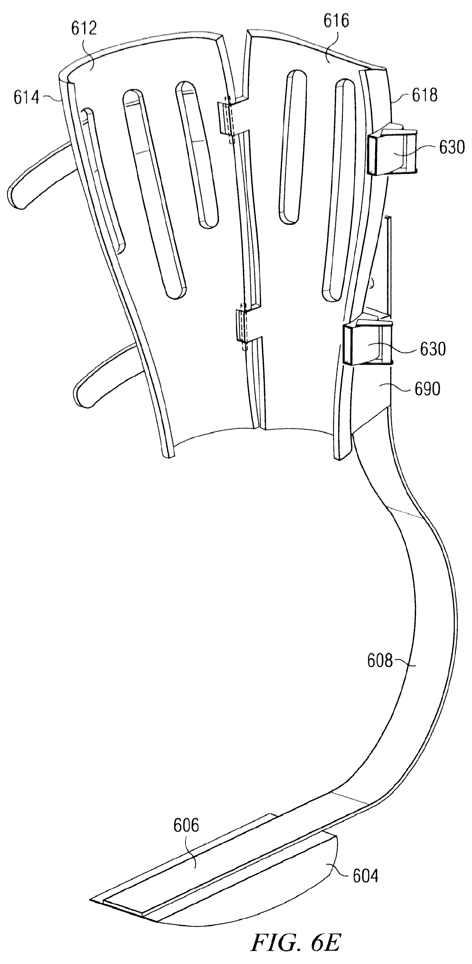

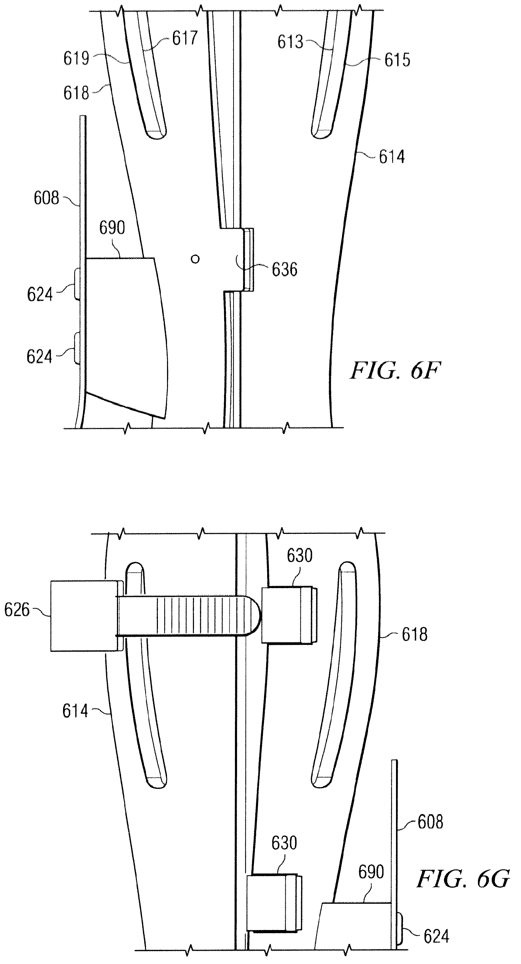

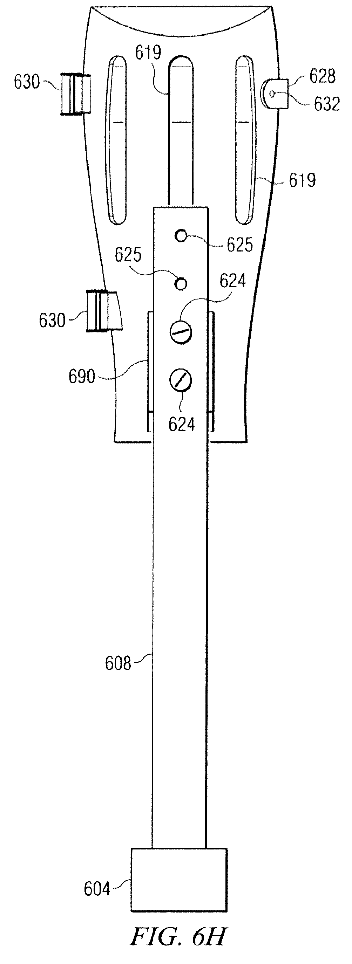

FIGURE 6A illustrates a generally isometric view of a device for ambulation of a subject having an impaired lower extremity according to a specific example embodiment of the disclosure;

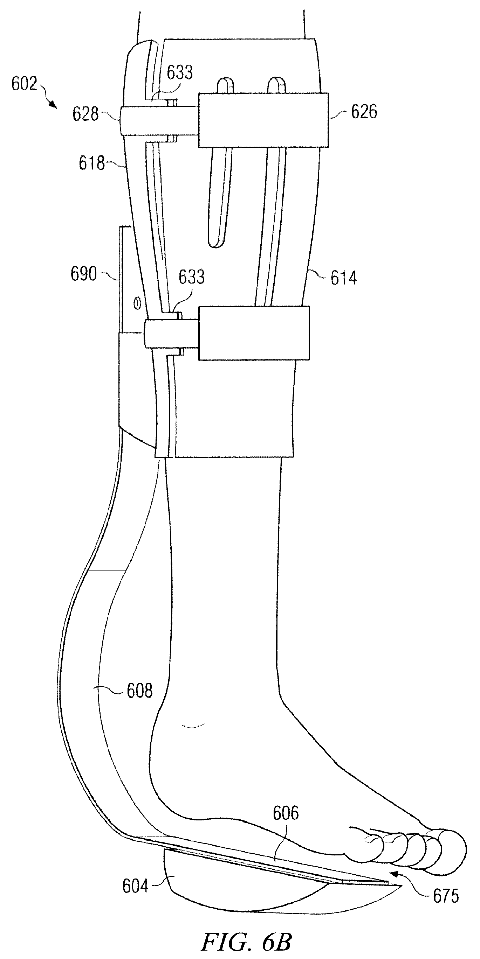

FIGURE 6B illustrates a generally isometric view of the device for ambulation of a subject shown in FIGURE 6A according to a specific example embodiment of the disclosure;

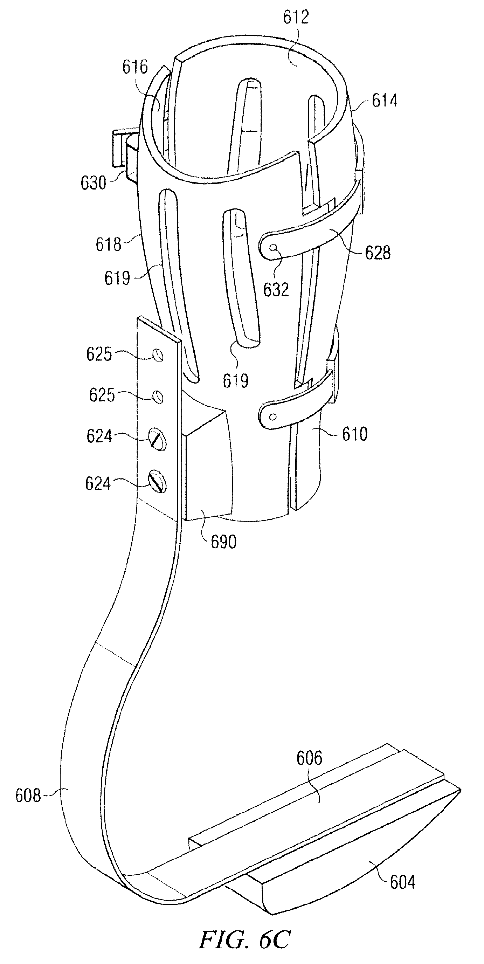

FIGURE 6C illustrates a generally isometric view of the device for ambulation of a subject shown in FIGURE 6A according to a specific example embodiment of the disclosure;

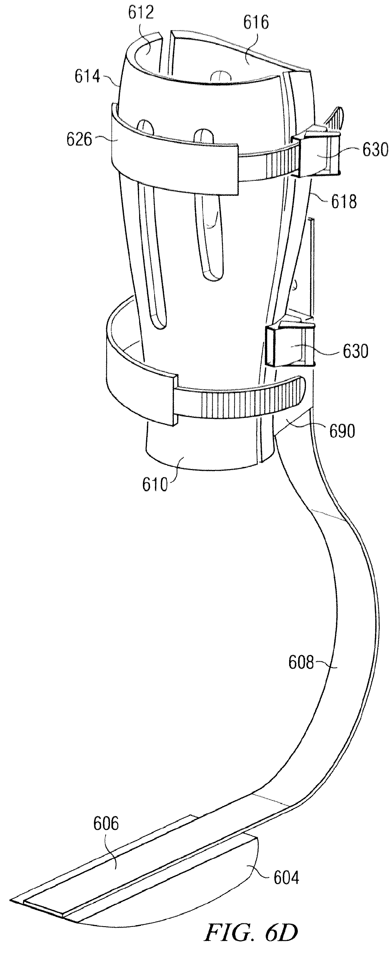

FIGURE 6D illustrates a generally isometric view of the device for ambulation of a subject shown in FIGURE 6A according to a specific example embodiment of the disclosure;

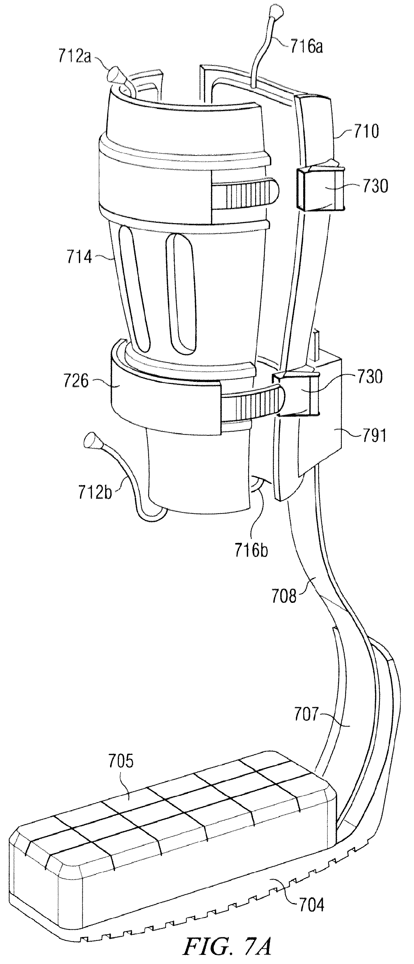

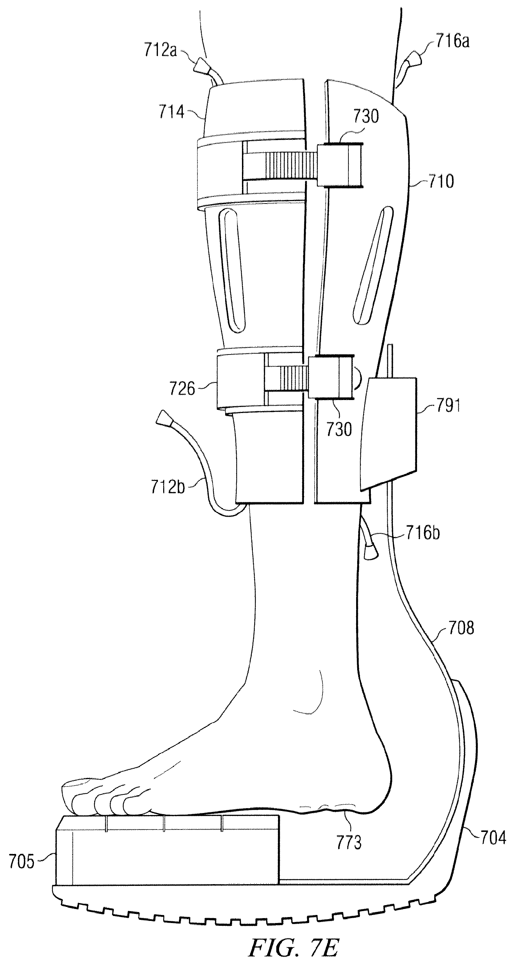

FIGURE 7E illustrates a left profile view of the device for ambulation of a subject shown in FIGURE 7A according to a specific example embodiment of the disclosure;

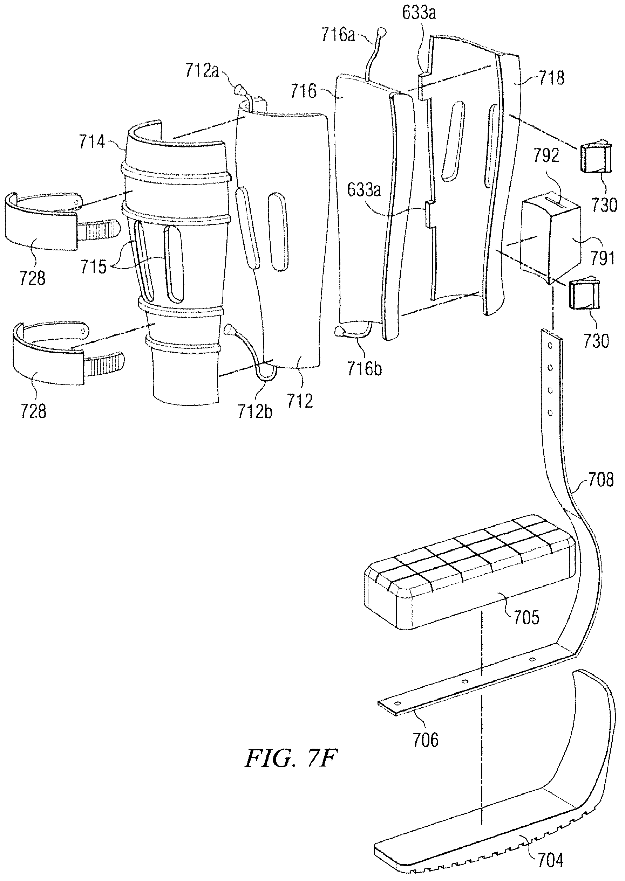

FIGURE 7F illustrates an exploded view of the device for ambulation of a subject shown in FIGURE 7A according to a specific example embodiment of the disclosure;

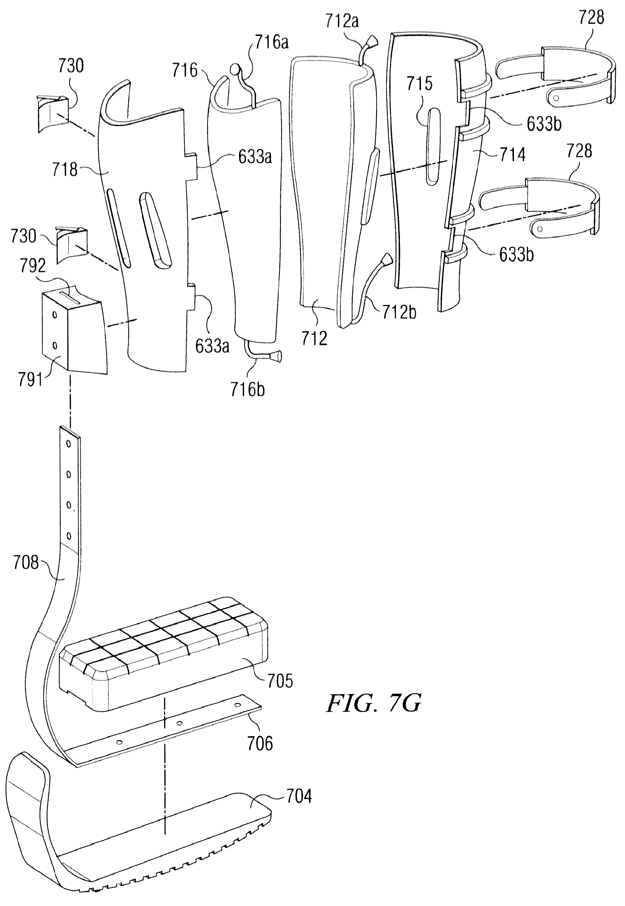

FIGURE 7G illustrates an exploded view of the device for ambulation of a subject shown in FIGURE 7A according to a specific example embodiment of the disclosure; and

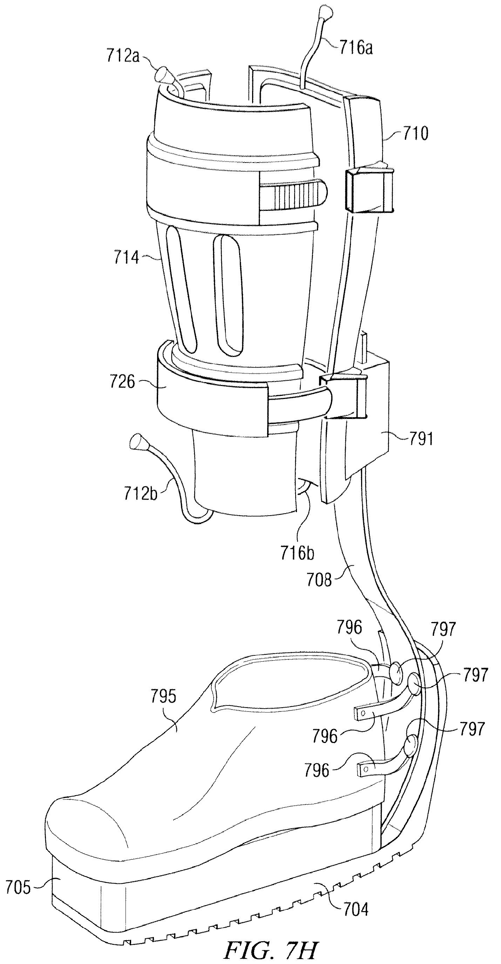

FIGURE 7H illustrates a generally isometric view of the device for ambulation of a subject shown in FIGURE 7A according to a specific example embodiment of the disclosure.

The present invention relates, according to some embodiments, to methods, devices, and systems for facilitating ambulation (*e.g.*, walking, running, dancing, and the like) of a subject having an impaired lower extremity. For example, a system for facilitating ambulation of a subject may include a walking brace configured to suspend a subject's lower extremity (*e.g.*, foot) in a non-weight-bearing position. In accordance with the invention, a system may transfer the subject's weight to at least a portion of the subject's lower leg and/or to at least a portion of the subject's upper leg. A weight-bearing portion of the lower leg may have a vertical span from about the gastrocnemius/solius muscles to about the tibial plateau. A weight-bearing portion of the upper leg may have a vertical span from just superior to the patella to about the proximal one third of the quadriceps femoris. A lower cuff may span up to the entire circumference of a subject's lower leg. An upper cuff may span up to the entire circumference of a subject's upper leg. When positioned upright, a subject's foot may dangle loosely. The subject's impaired lower extremity may have a wound dressing, a cast, a wrap, or other dressings or garments. With respect to a cast (and/or any other rigid and/or semi rigid material), care may be taken to ensure that the cast does not bear any weight since that load may be transmitted to a subject's foot. For example, a device may be adapted to accommodate a subject wearing a cast that covers the subject's ankle and extends up to about the mid-calf region (*e.g.,* by shortening the lower cuff by an amount sufficient to ensure that weight is not transferred from the brace to the cast). It may be desirable, in some embodiments, to include a covering and/or sling to protect and support a lower extremity. The choice of covering and/or sling may be influenced by the nature of the impairment and/or the nature of the dressing.

For example, it may be desirable to support and/or immobilize a subject's foot in a position that is generally perpendicular to the axis of the lower leg (*e.g.*, to prevent and/or minimize Achilles tendon contracture). For example, a sling attached (e.g., by a slide or spring to avoid permitting the foot to bear weight) to at least one cuff and/or at least one strut may be used to secure a subject's foot in this position. Alternatively, a platform may be fitted with one or more pads on its upper surface with the pad positioned, for example, under the ball of the foot or over the entire platform. A foot pad, in some embodiments, may be scored or perforated into segments to allow a health care worker or a subject to customize the support as needed. For example, it may be desirable to minimize Achilles tendon contracture in a subject with an open sore (e.g., diabetic foot ulcer) by providing foot support that does not contact the open sore. Segments of the pad may be selectively removed to avoid that contact.

According to some embodiments, an impairment of a lower extremity may include a foot impairment, an ankle impairment, and/or a knee impairment. An impairment may affect a subject's ability to bear weight on or near the effected region. For example, an impairment may exist where the pathology itself impedes the weight bearing capacity. An impairment may also exist where therapy, convalescence, and/or rehabilitation include relieving the region of weigh-bearing forces. Examples of lower extremity impairments may include, without limitation, a fractured bone, a broken bone, a sprain, an ulcer (*e.g.,* a diabetic ulcer), arthritis, a joint dislocation, a joint subluxation, a torn ligament (*e.g.,* a torn anterior cruciate ligament), a torn cartilage (*e.g.*, a torn meniscus), bursitis, tendonitis, an infection, gout, gangrene, plantar fasciitis, metabolic diseases, bone or cartilage diseases, neuropathic states and/or post-operative states.

In some embodiments, increased ambulation may reduce, eliminate, and/or prevent one or more conditions, including, for example, deep venous thrombosis, atrophy, pain (*e.g.,* lower back pain due to bed rest), and/or osteoporosis or other bone loss. Increasing a subject's ambulation without crutches may decrease the incidence of injuries associated with falling and/or upper body strain.

A load redistribution system, in accordance with the invention, includes a platform a single vertical strut fixed to the platform, and at least one cuff fixed to the at least one vertical strut. In some embodiments, one or more fasteners may be used to secure a load redistribution system to a subject's leg including, for example, belts, buckles (*e.g.*, ski boot buckles, ratcheting buckles), buttons, cams, carabineers, chains, cinches, clasps, D-rings, draw-strings, hooks, levers, locks, loops, slides, snaps, straps, and/or tensioners. These fasteners may be attached to the at least one vertical strut and/or the at least one cuff.

A load redistribution system in accordance with the invention is configured to bear and/or redistribute a load (*e.g.,* a subject's weight). For example, a load redistribution system may be configured to transfer the load of s subject's weight away from the subject's foot and transfer it to the subject's lower and/or upper leg. A load redistribution system may comprise one or more struts positioned adjacent to a subject's leg (*e.g.,* in front of, behind, to the right, of and/or to the left of a subject's leg). For example, a single strut may be positioned on the left side of a left leg, on the right side of a right leg, or behind a left leg or right leg. In some embodiments, a strut may be positioned on the generally opposite side of a leg from a subject's sagittal plane or on the generally opposite side of a leg from a subject's coronal plane. In some embodiments, a strut positioned generally behind the subject's leg on which the system is worn may have an S-curve profile. For example, a strut may be configured to have a profile that resembles a letter "S" in that it has two opposing inflection points (*e.g.*, one anterior and one posterior). An S-curve profile may permit clearance for a subject's foot and/or a normal stride.

A strut may comprise, according to some embodiments, two pieces (*e.g.,* an upper piece and a lower piece) joined by a hinge. In some embodiments, a hinge may be configured to permit a limited range (*e.g.,* limited flexion and/or extension) and/or an unlimited range of rotational motion. According to some embodiments, a two-strut system may have a strut on each side of a subject's leg, with each strut having a single piece (*e.g.,* lower leg only) or two pieces connected by a hinge (*e.g.,* a full leg brace).

In accordance with the invention, a load redistribution system is configured to bear a load. A load may include, for example, a subject's weight, and/or a multiple of a subject's weight (*e.g.*, 1.1x, 1.2x, 1.5x, and/or 2x). It may be desirable for a load redistribution system to bear a more than a subject's weight, for example, where the subject may carry additional weight (*e.g.,* a backpack, a bag of groceries, a child, and/or the like) and/or may put the system under additional stress (*e.g.*, through sport, exercise, or the like). In some embodiments, a subject's lower extremity (*e.g.*, foot) with a load redistribution system in place bears little or no weight, even though weight is born by that leg (*e.g.,* while standing, exercising, working, and/or engaging in other activities).

A load redistribution system may include at least one cuff fixed to at least one strut in some embodiments. For example, a system may include a lower leg cuff and an upper leg cuff. A system may include a plurality of lower cuffs and/or a plurality of upper cuffs in some embodiments. For example, if a single cuff would contact and/or cover an injured portion of a lower leg, it may be desirable to instead use two or more lower cuffs configured to minimize and/or avoid contacting/covering the effected region. A cuff may be fixedly or adjustably mounted to a corresponding strut in some embodiments. For example, a strut may include a series of holes (*e.g.*, equidistantly spaced) configured to receive a corresponding pin (*e.g.*, spring-loaded) and/or screw attached to a cuff. A user may slide the cuff along the length of a strut and engage the screw and/or pin when a desired position is found. In some embodiments, a load redistribution system may include continuously variable adjustment system. A cuff may slide along the length of a strut with one or more set screws configured to fix the relative positions of each once a desirable position is found.

In some embodiments, a cuff may include a pad disposed to contact at least a portion of a subject's leg and an outer shell. An inner pad may comprise any desirable gel, foam, and/or other cushioning material(s). A custom fit cuff may facilitate, according to some embodiments, uniform distribution (*e.g.*, uniform, substantially uniform, relatively uniform) of a subject's weight over the surface area of the cuff. An outer shell may comprise a moldable material.

In some embodiments, a cuff may be opened on at least one side to facilitate donning and/or removing the cuff (and attached load redistribution system). For example, a cuff may comprise two outer shells with one or more hinges arranged on one side to allow the cuff to open clamshell-style. Two or more outer shells may fit together without a hinge or other permanent connection according to some embodiments. Once donned, a cuff may be closed and/or secured using any type of fixed or adjustable tensioning system. For example, one or more collars, straps, cinches, guides, loops, hooks, hoops, buckles, and/or combinations thereof may be used. In some embodiments, a tensioning system may include hooks and/or loops (*e.g.*, VELCRO®).

According to some embodiments, a load redistribution system, when donned, will increase a subject's inseam. This may result in an undesirable difference between the inseam of the leg on which a load redistribution system is worn and the inseam of the free leg. Any difference may be offset, at least partially, by wearing a lift (*e.g.,* integrated and/or inserted in a shoe) or other apparatus on the free leg.

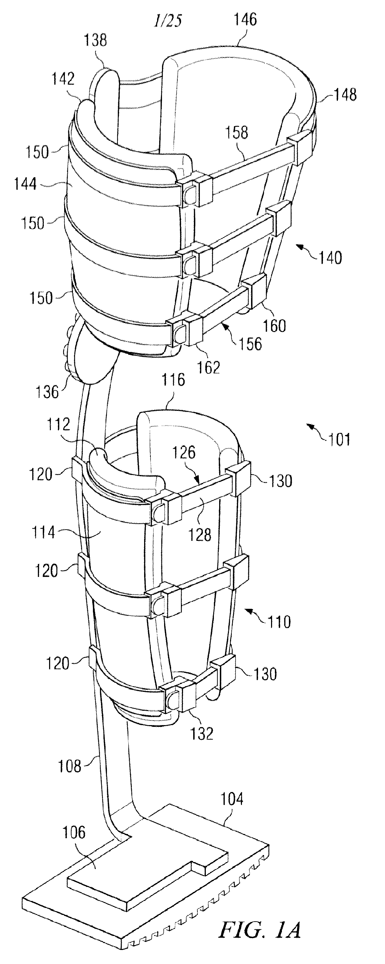

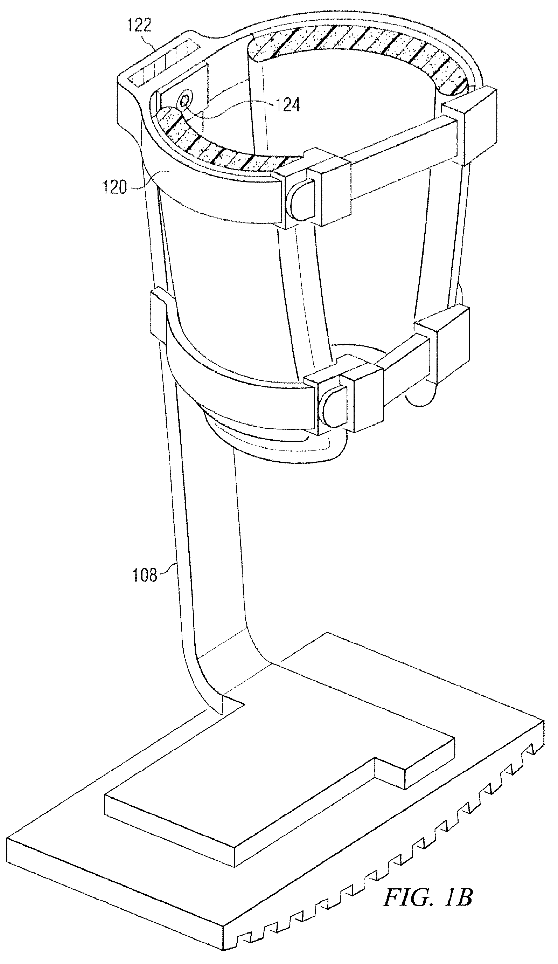

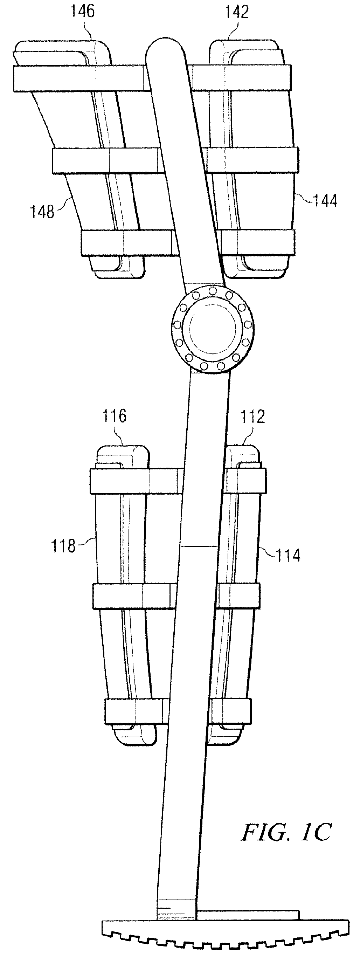

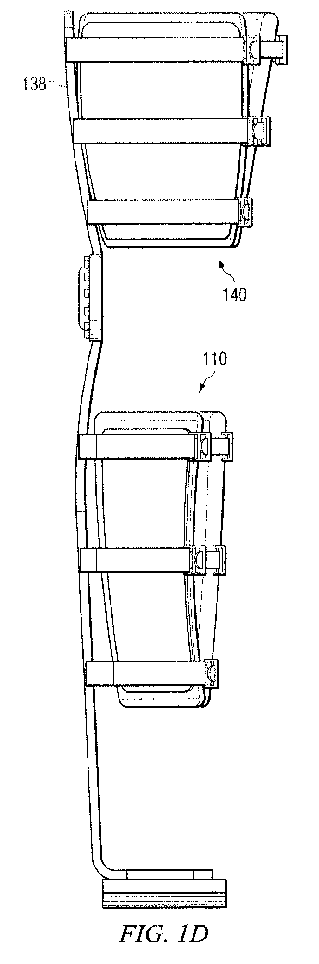

FIGURES 1A-1D illustrate an example of a load redistribution system **101** for ambulation of a subject having a lower extremity impairment, in accordance with embodiments of the present disclosure. As shown in FIGURES 1A-1D, load redistribution system **101** may include outsole **104,** platform **106,** lower strut **108,** lower cuff **110,** hinge **136,** upper strut **138,** and upper cuff **140.**

Outsole **104** may comprise a foot pad, the bottom of which may be rounded (*e.g.,* rocker outsole) to facilitate walking. The bottom may be ridged as pictured or may have another tread pattern to aid ambulation. Outsole **104** may comprise a hard rubber or other suitable material. Outsole **104** may be generally rectangular in shape as depicted. Other regular and/or irregular shapes may be suitable and/or desirable in some embodiments. Platform **106** may be contiguous with lower strut **108** as shown or may be a separate piece fixedly attached (*e.g.*, welded, bolted) to lower strut **108.** In some embodiments, outsole **104** and platform **106** may be adjustable (*e.g.*, fore and aft) relative to each other. The lower surface of platform **106** may be affixed to outsole **104** with any type of fastener and/or adhesive. Platform **106** may sit atop outsole **104** as shown. In some embodiments, platform **106** may be recessed within outsole **104,** for example, so that the upper surface of outsole **104** is flush with the upper surface of platform **106.** Platform **106** may have any regular or irregular shape. Platform **106** may be somewhat smaller than outsole **104** as depicted or may be sized to match the size of outsole **104.** In some embodiments, it may be desirable for platform **106** to be larger than outsole **104.** Platform **106** may comprise a rigid material suitable for bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

Lower strut **108** may extend vertically from platform **106** to hinge **136.** Upper strut **138** may extend vertically from hinge **136** to a position corresponding to or just below a subject's hip. Lower strut **108** may have a lengthwise axis that is approximately parallel to the lengthwise axis of a subject's leg (*e.g.,* lower leg). Upper strut **138** may have a lengthwise axis that is approximately parallel to the lengthwise axis of a subject's leg (*e.g.,* upper leg). Lower strut **108** and/or upper strut **138** may comprise one or more rigid materials capable of bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

As depicted, lower cuff **110** may comprise anterior pad **112,** anterior pad shell **114,** posterior pad **116,** posterior pad shell **118,** calf collar **120,** and tension adjustment fastener **126.** Pads **112** and/or **116** may independently comprise flexible, elastomeric, and/or resilient materials including, for example, polyurethane, polyethylene, neoprene, ethylene vinyl acetate, foam, silicone, rubber, and the like. Pad shells **114** and/or **118** may independently comprise a moldable plastic. In some embodiments, pad shells **114** and/or **118** may independently comprise steel, a steel alloy, aluminum, an aluminum alloy, titanium, a titanium alloy, carbon fiber, an aramid fiber, a para-aramid fiber (*e.g.*, KEVLAR), fiberglass, and combinations thereof. Pad shells **114** and/or **118** comprising a moldable material may be custom fit to a subject. A moldable plastic may include, for example, a thermoplastic (*e.g.*, capable of more than one cycle of melting, molding, and setting) and/or a thermosetting plastic (e.g., capable of one cycle of melting, molding, and setting). Pads **112** and/or **116** may be adhered, welded, bonded, stitched, or otherwise fixed to pad shells **114** and/or **118,** respectively.

Pad shells **114** and/or **118** may be fixedly attached to calf collar **120.** Calf collar **120** may extend up to all the way around the circumference of a subject's lower leg. Calf collar **120** may include sleeve **122** positioned, for example, at or near its midpoint. Sleeve **122** may be configured to receive strut **108.** Sleeve **122** may have a cavity, the longitudinal axis of which is about perpendicular to the lengthwise axis of strut **108.** Sleeve **122** may be configured to allow collar **120** to slide along the length of strut **108.** Sleeve **122** may include set screw **124** positioned to adjustably contact strut **108.** Each calf collar may be configured to include a sleeve and each sleeve may include a set screw. In use, set screw **124** may be loosened to permit collar **120** to slide along the length of strut **108.** Once a desirable position is found, set screw **124** may be tightened to fix the position of collar **120** on strut **108.** Set screw **124** may be positioned on the inside of collar **120** and directed outwardly toward strut **108** as shown. In some embodiments, set screw **124** may be positioned on the outside of collar **120** and directed inwardly toward strut **108.** As depicted, lower cuff **110** may include three calf collars **120.** In some embodiments, more or fewer collars may be desired and/or required.

Tension adjustment fastener **126** may include strap **128,** cinch **130,** and anchor **132.** Strap **128** maybe fixed to one end of calf collar **120** via anchor **132.** Cinch **130** may be fixed to the other end of collar **120.** Cinch **130** may be configured to receive and releasably grip strap **128.** In use, strap **128** may be threaded through cinch **130** and pulled tight to apply a desired amount of pressure on the subject's lower leg. Cinch **130** may then be closed to fix strap **128** in its position. As depicted, the loose end of strap **120** may be tucked into anchor **132.**

Hinge **136** may link lower strut **108** and upper strut **138** and permit rotation of lower strut **108** and upper strut **138** relative to each other. When system **101** is in position on a subject's leg, the hinge axis of hinge **136** may be parallel or substantially parallel to the hinge axis of the subject's knee in good health. It may be desirable, in some embodiments, to configure hinge **136** to have a limited and/or selectable degree of rotation (*e.g.*, flexion and/or extension).

As depicted, upper cuff **140** may comprise anterior pad **142,** anterior pad shell **144,** posterior pad **146,** posterior pad shell **148,** thigh collar **150,** and tension adjustment fastener **156.** Pads **142** and/or **146** may independently comprise flexible, elastomeric, and/or resilient materials including, for example, polyurethane, polyethylene, neoprene, ethylene vinyl acetate, foam, silicone, rubber, and the like.

In some embodiments, pad shells **144** and/or **148** may independently comprise a moldable plastic, steel, a steel alloy, aluminum, an aluminum alloy, titanium, a titanium alloy, carbon fiber, an aramid fiber, a para-aramid fiber (*e.g.*, KEVLAR), fiberglass, and combinations thereof. Pad shells **144** and/or **148** comprising a moldable material may be custom fit to a subject. A moldable plastic may include, for example, a thermoplastic (*e.g.*, capable of more than one cycle of melting, molding, and setting) and/or a thermosetting plastic (*e.g.*, capable of one cycle of melting, molding, and setting). Pads **142** and/or **146** may be adhered, welded, bonded, stitched, or otherwise fixed to pad shells **144** and/or **148,** respectively.

Pad shells **144** and/or **148** may be fixedly attached to thigh collar **150.** Thigh collar **150** may extend up to all the way around the circumference of a subject's upper leg. Thigh collar **150** may include sleeve **152** positioned, for example, at or near its midpoint. Sleeve **152** may be configured to receive strut **138.** Sleeve **152** may have a cavity, the longitudinal axis of which is about perpendicular to the lengthwise axis of strut **138.** Sleeve **152** may be configured to allow collar **150** to slide along the length of strut **138.** Sleeve **152** may include set screw **154** positioned to adjustably contact strut **138.** In use, set screw **154** may be loosened to permit collar **150** to slide along the length of strut **138.** Once a desirable position is found, set screw **154** may be tightened to fix the position of collar **150** on strut **138.** Set screw **154** may be positioned on the inside of collar **150** and directed outwardly toward strut **138** as shown. In some embodiments, set screw **154** may be positioned on the outside of collar **150** and directed inwardly toward strut **138.** As depicted, upper cuff **140** may include three thigh collars **150.** In some embodiments, more or fewer collars may be desired and/or required.

Tension adjustment fastener **156** may include strap **158,** cinch **160,** and anchor **162.** Strap **158** maybe fixed to one end of thigh collar **150** via anchor **162.** Cinch **160** may be fixed to the other end of collar **150.** Cinch **160** may be configured to receive and releasably grip strap **158.** In use, strap **158** may be threaded through cinch **160** and pulled tight to apply a desired amount of pressure on the subject's upper leg. Cinch **160** may then be closed to fix strap **158** in its position. As depicted, the loose end of strap **150** may be tucked into anchor **162.**

FIGURES 2A-2B illustrate an example load redistribution system **201** for ambulation of a subject having a lower extremity impairment, in accordance with embodiments of the present disclosure. As shown in FIGURES 2A-2B, system **201** may be configured (*e.g.,* formed and/or adjusted) to include gap **275** between a subject's foot **272** and the higher of outsole **204** and platform **206.** Gap **275** relieves subject's foot **272** and/or other lower extremities from bearing weight by diverting the load elsewhere. In some embodiments, gap **275** may be any suitable distance that avoids (*e.g.*, minimizes, eliminates) loading foot **272** and/or other lower extremities. Gap **275** may depend, in part, upon the size and weight of the subject user. For example, a subject with a long foot may be inclined and/or compelled to support some weight on foot **272** at some points in a walking cycle (*e.g.*, when foot **272** is at it's most rearward position). It may be desirable in such situations to adjust gap **275** to be larger. A subject, for example, a smaller subject, may desire and/or require a smaller gap **675,** for example, to reduce or minimize the difference in length between the effected leg with system **602** and a healthy leg. In some embodiments, gap **275** may be from about 1 cm to about 5 cm, from about 1 cm to about 15 cm, from about 1 cm to about 10 cm, from about 1 cm to about 20 cm, from about 2 cm to about 5 cm, from about 2 cm to about 10 cm, from about 2 cm to about 15 cm, and/or from about 2 cm to about 20 cm. Gap **275** may be less than about 1 cm or more than 20 cm in some embodiments.

FIGURES 3A-3D illustrate an example of a load redistribution system **302** for ambulation of a subject having a lower extremity impairment, in accordance with embodiments of the present disclosure. As shown in FIGURES 3A-3D, load redistribution system **302** may include outsole **304,** platform **306,** lower strut **308,** and lower cuff **310.**

Outsole **304** may comprise a foot pad, the bottom of which may be rounded (*e.g.,* rocker outsole) to facilitate walking. The bottom may be ridged as pictured or may have another tread pattern to aid ambulation. Outsole **304** may comprise a hard rubber or other suitable material. Outsole **304** may be generally rectangular in shape as depicted. Other regular and/or irregular shapes may be suitable and/or desirable in some embodiments. Platform **306** may be contiguous with strut **308** as shown or may be a separate piece fixedly attached to strut **308.** The lower surface of platform **306** may be affixed to outsole **304** with any type of fastener and/or adhesive. Platform **306** may sit atop outsole **304** as shown. In some embodiments, platform **306** may be recessed within outsole **304,** for example, so that the upper surface of outsole **304** is flush with the upper surface of platform **306.** Platform **306** may have any regular or irregular shape. Platform **306** may be somewhat smaller than outsole **304** as depicted or may be sized to match the size of outsole **304.** In some embodiments, it may be desirable for platform **306** to be larger than outsole **304.** Platform **306** may comprise a rigid material suitable for bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

Strut **308** may extend vertically from platform **306** to hinge to a position corresponding to or just below a subject's tibia. Strut **308** may have a lengthwise axis that is approximately parallel to the lengthwise axis of a subject's leg (*e.g.,* lower leg). Strut **308** may comprise one or more rigid materials capable of bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

As depicted, lower cuff **310** may comprise anterior pad **312,** anterior pad shell **314,** posterior pad **316,** posterior pad shell **318,** calf collar **320,** and tension adjustment fastener **326.** Pads **312** and/or **316** may independently comprise flexible, elastomeric, and/or resilient materials including, for example, polyurethane, polyethylene, neoprene, ethylene vinyl acetate, foam, silicone, rubber, and the like. In some embodiments, pad shells **314** and/or **318** may independently comprise a moldable plastic, steel, a steel alloy, aluminum, an aluminum alloy, titanium, a titanium alloy, carbon fiber, an aramid fiber, a para-aramid fiber *(e.g.,* KEVLAR), fiberglass, and combinations thereof. Pad shells **314** and/or **318** comprising a moldable material may be custom fit to a subject. A moldable plastic may include, for example, a thermoplastic (*e.g.*, capable of more than one cycle of melting, molding, and setting) and/or a thermosetting plastic (*e.g.*, capable of one cycle of melting, molding, and setting). Pads **312** and/or **316** may be adhered, welded, bonded, stitched, or otherwise fixed to pad shells **314** and/or **318,** respectively.

Pad shells **314** and/or **318** may be fixedly attached to calf collar **320.** Calf collar **320** may extend up to all the way around the circumference of a subject's lower leg. Calf collar **320** may include sleeve **322** positioned, for example, at or near its midpoint. Sleeve **322** may be configured to receive strut **308.** Sleeve **322** may have a cavity, the longitudinal axis of which is about perpendicular to the lengthwise axis of strut **308.** Sleeve **322** may be configured to allow collar **320** to slide along the length of strut **308.** Sleeve **322** may include set screw **324** positioned to adjustably contact strut **308.** Each calf collar may be configured to include a sleeve and each sleeve may include a set screw. In use, set screw **324** may be loosened to permit collar **320** to slide along the length of strut **308.** Once a desirable position is found, set screw **324** may be tightened to fix the position of collar **320** on strut **308.** Set screw **324** may be positioned on the inside of collar **320** and directed outwardly toward strut **308** as shown. In some embodiments, set screw **324** may be positioned on the outside of collar **320** and directed inwardly toward strut **308.** As depicted, lower cuff **310** may include three calf collars **320.** In some embodiments, more or fewer collars may be desired and/or required.

Tension adjustment fastener **326** may include strap **328,** cinch **330,** and anchor **332.** Strap **328** maybe fixed to one end of calf collar **320** via anchor **332.** Cinch **330** may be fixed to the other end of collar **320.** Cinch **330** may be configured to receive and releasably grip strap **328.** In use, strap **328** may be threaded through cinch **330** and pulled tight to apply a desired amount of pressure on the subject's lower leg. Cinch **330** may then be closed to fix strap **328** in its position. As depicted, the loose end of strap **320** may be tucked into anchor **332.**

FIGURE 4 illustrates an example of a load redistribution system **403** for ambulation of a subject having a lower extremity impairment, not in accordance with embodiments of the present invention. As shown in FIGURE 4, load redistribution system **403** may include lower cuff **410,** upper cuff **440,** and rod assembly **480.**

Lower cuff **410,** as shown, may include anterior pad **412,** anterior pad shell **414,** calf collar **420,** and tension adjustment fastener **426.** Lower cuff **410** may be secured on a subject's lower leg in a manner similar to cuffs **110** and **310.** Upper cuff **440,** as shown, may include, posterior pad **446,** posterior pad shell **448,** calf collar **450,** and tension adjustment fastener **456.** Upper cuff **440** may be secured on a subject's upper leg in a manner similar to cuffs **140** and **340.** Rod assembly **480,** as shown, may include foot **482,** vertical rod **484,** and lateral bar **486.**

Lateral bar **486** may be fixed to lower cuff **410** at one end and fixed to vertical rod **484** at the other as shown. In some embodiments, a lower cuff may be fixed directly to a vertical rod allowing the lateral bar to be omitted. Upper cuff **440** may be fixed directly to vertical rod **484.** In some embodiments, a lateral bar may be interposed between an upper cuff and a vertical rod. Rod assembly **480** may comprise one or more rigid materials capable of bearing a subject's weight. Examples of such materials include, without limitation, steel, a steel alloy, aluminum, an aluminum alloy, titanium, a titanium alloy, carbon fiber, wood, fiberglass and combinations thereof.

Variations in the weight of a subject, the positioning of the cuff(s), and/or the dimensions of the cuff(s) may influence the pressure on a subject's leg. In some embodiments, the pressure applied by a cuff on a subject's leg may be up to about 34.5 kPa, up to about 51.7 kPa, up to about 68.9 kPa, up to about 103.4 kPa, up to about 137.9 kPa, up to about 172.4 kPa, and/or up to about 206.8 kPa (about 5 psi, up to about 7.5 psi, up to about 10 psi, up to about 15 psi, up to about 20 psi, up to about 25 psi, and/or up to about 30 psi).

In some embodiments, a system may include one or more sensors (*e.g.*, pressure sensors). One or more sensors configured to detect the pressure at a particular location and/or the load born and/or or shifted

FIGURES 5A and 5B illustrate an example of a load redistribution system **501** for ambulation of a subject having a lower extremity impairment, in accordance with embodiments of the present disclosure. As shown in FIGURE 5A, load redistribution system **501** may support a subject's weight through lower strut **508** (not expressly shown), lower cuff **510** (comprising circumferential pad **511**), upper strut **538** (not expressly shown), and upper cuff **540** (comprising circumferential pad **541**). FIGURE 5A illustrates a method for calculating the pressure to be applied to a subject's leg as system **501** bears a subject's weight. Subject's weight is represented as vector V. As depicted, pads **511** and **541** are positioned at an angle $\theta$ relative to vector V. The force applied to pad **511** is represented as vector $F_{P1}$ and the force applied to pad **511** is represented as vector $F_{P2}$. The horizontal component of vector $F_{P1}$ is represented by vector $F_{X1}$ and the vertical component is represented by vector $F_{Y1}$. The horizontal component of vector $F_{P2}$ is represented by vector $F_{X2}$ and the vertical component is represented by vector $F_{Y2}$. Since system **501** is configured to bear a subject's full weight (w),

$$w = F_{Y1} + F_{Y2} \qquad\qquad \text{(Equation 1)}$$

If the subject's weight (w) is distributed evenly between pads **511** and **541,**

$$w/2 = F_{Y1} = F_{Y2} \qquad\qquad \text{(Equation 2)}$$

Thus, since

$$\sin(\theta) = F_{Y1} / F_{P1} \qquad\qquad \text{(Equation 3)}$$

the force $F_{P1}$ applied to a subject's leg may be calculated as follows:

$$F_{P1} = F_{Y1} / \sin(\theta) \qquad\qquad \text{(Equation 4)}$$

The area of cuff **510** is given by Equation 5:

$$\text{Area} = [(d_1 + d_2)/2] * L * \pi \qquad\qquad \text{(Equation 5)}$$

Thus, the pressure (P) on a subject's leg at cuff **510** is given by Equation 4:

$$P = \frac{F_{Y1} / \sin(\theta)}{[(d_1 + d_2)/2] * L * \pi} \qquad\qquad \text{(Equation 6)}$$

Therefore, if pad **511** is positioned at an angle of 7° ($\theta$), $d_1$ is 13.74 cm (5.41 inches), $d_2$ is 8.89 cm (3.5 inches), L is 17.78 cm (7 inches), and the subject's weight is 81.6 kg (180 pounds) the pressure (P) is given by

$$P = \frac{(w/2) / \sin(\theta)}{[(d_1 + d_2)/2] * L * \pi} \qquad\qquad \text{(Equation 7)}$$

$$P = \frac{(180/2) \,/\, \sin(7)}{[(5.41 + 3.5)/2] * 7 * \pi} \qquad \text{(Equation 8)}$$

$$P = \quad 51.7 \text{ kPa } (7.5 \text{ psi})$$

FIGURES 6A-6H illustrate an example of a load redistribution system **602** for ambulation of a subject having a lower extremity impairment, in accordance with embodiments of the present disclosure. As shown in FIGURES 6A-6H, load redistribution system **602** may include outsole **604**, platform **606**, strut **608**, support **690**, and cuff **610**.

Outsole **604** may comprise a foot pad, the bottom of which may be rounded (*e.g.,* rocker outsole) to facilitate walking. The bottom may be ridged as pictured or may have another tread pattern to aid ambulation. Outsole **604** may comprise a hard rubber or other suitable material. Outsole **604** may be generally rectangular in shape as depicted. Other regular and/or irregular shapes may be suitable and/or desirable in some embodiments. Platform **606** may be contiguous with strut **608** as shown or may be a separate piece fixedly attached to strut **608**. In some embodiments, outsole **604** and platform **606** may be adjustable (*e.g.*, fore and aft) relative to each other. The lower surface of platform **606** may be affixed to outsole **604** with any type of fastener and/or adhesive. Platform **606** may sit atop outsole **604** as shown. In some embodiments, platform **606** may be recessed within outsole **604**, for example, so that the upper surface of outsole **604** is flush with the upper surface of platform **606**. Platform **606** may have any regular or irregular shape. Platform **606** may be somewhat smaller than outsole **604** as depicted or may be sized to match the size of outsole **604**. In some embodiments, it may be desirable for platform **606** to be larger than outsole **604**. Platform **606** may comprise a rigid material suitable for bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

System **602** may include strut **608** positioned substantially behind a subject's leg when the leg is secured in system **602**. At least a portion of strut **608** may be fixedly connected to and/or contiguous with a posterior portion of platform **606**. At least a portion of strut **608** extends from a posterior portion of platform **606** in the same plane as platform **606** or a plane generally parallel to the plane of platform **606** ("horizontal" portion). At least a portion of strut **608** rises above the plane of platform **606** and generally perpendicular to the plane of platform **606** ("vertical" portion). This vertical portion may have a lengthwise axis that is approximately parallel to the lengthwise axis of a subject's leg (*e.g.,* lower leg). Strut **608** may include bend **607** interposed between the horizontal and vertical portions of strut **608**. Bend **607** may be configured to bend or curve upward from platform **606** in a manner that permits a subject a normal or generally normal stride (*e.g.,* heel to toe contact with the ground). Strut **608** may comprise one or more rigid materials capable of bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof. While strut **608** may be rigid to support a subject's weight, some resilience/flexibility may be tolerated and/or desirable in some embodiments.

As depicted, cuff **610** may comprise anterior pad **612**, anterior pad shell **614**, posterior pad **616**, posterior pad shell **618**, and tension adjustment fastener **626**. Pad **612** may include one or more apertures **613**. Shell **614** may include one or more apertures **615**. Pad **616** may include one or more apertures **617**. Shell **618** may include one or more apertures **619**. Pad apertures **613** and **617** may be independently aligned (*e.g.*, partially or completely) with shell apertures **615** and **619**, respectively. Pads **612** and/or **616** may independently comprise flexible, elastomeric, and/or resilient materials including, for example, polyurethane, polyethylene, neoprene, ethylene vinyl acetate, foam, silicone, rubber, and the like. Pad shells **614** and/or **618** may independently comprise a moldable plastic. In some embodiments, pad shells **614** and/or **618** may independently comprise steel, a steel alloy, aluminum, an aluminum alloy, titanium, a titanium alloy, carbon fiber, an aramid fiber, a para-aramid fiber (*e.g.*, KEVLAR), fiberglass, and combinations thereof. Pad shells **614** and/or **618** comprising a moldable material may be custom fit to a subject. A moldable plastic may include, for example, a thermoplastic (*e.g.*, capable of more than one cycle of melting, molding, and setting) and/or a thermosetting plastic (*e.g.*, capable of one cycle of melting, molding, and setting). Pads **612** and/or **616** may be adhered, welded, bonded, stitched, or otherwise fixed to pad shells **614** and/or **618**, respectively.

As shown in FIGURE 6C, pad shell **618** may be fixedly attached to support **690**. Pad shells **614** and **618** may be attached to each other through one or more hinges **633**. Support **690** may be attached to strut **608** - one or more screws **624** may be secured to support **690** through one or more holes **625** in strut **608**. As shown in FIGURE 6C, a plurality of holes **625** along a vertical axis of strut **608** affords a health care worker and/or a subject the opportunity to adjust the height of cuff **610**.

Tension adjustment fastener **626** may include strap **628**, cinch **630**, and anchor **632**. Strap **628** maybe fixed to posterior pad shell **618** via anchor **632**. Cinch **630** may be fixed to posterior pad shell **618** at a point along the circumference of shell **618** and spaced away from anchor **632**. Cinch **630** may be configured to receive and releasably grip strap **628**. In use, strap **628** may be threaded through cinch **630** and pulled tight to apply a desired amount of pressure on the subject's lower leg. Cinch **630** may then be closed to fix strap **628** in its position. System **602** may include one, two (pictured), three or more tension adjustment fasteners **626**.

FIGURE 6B illustrates an example load redistribution system **602** for ambulation of a subject having a lower extremity impairment, in accordance with embodiments of the present disclosure. As shown in FIGURE 6B, system **602** may be configured (e.g., formed and/or adjusted) to include gap **675** between a subject's foot **672** and the higher of outsole **604** and platform **606**. Gap **675** relieves subject's foot **672** and/or other lower extremities from bearing weight by diverting at least a portion of the load elsewhere (e.g., all or substantially all of the load). In some embodiments, gap **675** may be any suitable distance that avoids (e.g., minimizes, eliminates) loading foot **672** and/or other lower extremities. The extent of gap **675** may depend, in part, upon the size and weight of the subject user. For example, a subject with a long foot may be inclined and/or compelled to support some weight on foot **672** at some points in a walking cycle (e.g., when foot **672** is at it's most rearward position with the toes nearest the ground and the heal elevated). It may be desirable in such situations to adjust gap **675** to be larger. A subject, for example, a smaller subject, may desire and/or require a smaller gap **675**, for example, to reduce or minimize the difference in length between the effected leg with system **602** and a healthy leg. In some embodiments, gap **675** may be from about 1 cm to about 5 cm, from about 1 cm to about 15 cm, from about 1 cm to about 10 cm, from about 1 cm to about 20 cm, from about 2 cm to about 5 cm, from about 2 cm to about 10 cm, from about 2 cm to about 15 cm, and/or from about 2 cm to about 20 cm. Gap **675** may be less than about 1 cm or more than 20 cm in some embodiments.

FIGURES 7A-7H illustrate an example of a load redistribution system **702** for ambulation of a subject having a lower extremity impairment, in accordance with embodiments of the present disclosure. As shown in FIGURES 7A-7H, load redistribution system **702** may include outsole **704**, pad **705**, platform **706**, strut **708**, support **791**, and cuff **710**.

Outsole **704** may comprise a foot pad, the bottom of which may be rounded (e.g., rocker outsole) to facilitate walking. The bottom may be ridged as pictured or may have another tread pattern to aid ambulation. Outsole **704** may comprise a hard rubber or other suitable material. Outsole **704** may have any regular or irregular shape. For example, outsole **704** may be configured to resemble the tread of a running shoe, a dress shoe, and/or a boot. As depicted, outsole **704** may include a flange that extends from the plane of the walking surface in a generally vertical direction along the back of strut **708**. This flange may facilitate traction and/or a normal or improved stride. Other regular and/or irregular shapes may be suitable and/or desirable in some embodiments. Platform **706** may be contiguous with strut **708** as shown or may be a separate piece fixedly attached to strut **708**. In some embodiments, outsole **704** and platform **706** may be adjustable (e.g., fore and aft) relative to each other. The lower surface of platform **706** may be affixed to outsole **704** with any type of fastener and/or adhesive. Platform **706** may sit atop outsole **704** as shown. In some embodiments, platform **706** may be recessed within outsole **704**, for example, so that the upper surface of outsole **704** is flush with the upper surface of platform **706**. Platform **706** may have any regular or irregular shape. Platform **706** may be somewhat smaller than outsole **704** as depicted or may be sized to match the size of outsole **704**. In some embodiments, it may be desirable for platform **706** to be larger than outsole **704**. Platform **706** may comprise a rigid material suitable for bearing a subject's weight. Examples of such materials include, without limitation, steel, a steel alloy, aluminum, an aluminum alloy, titanium, a titanium alloy, carbon fiber, wood, an aramid fiber, a para-aramid fiber (e.g., KEVLAR), fiberglass, and combinations thereof.

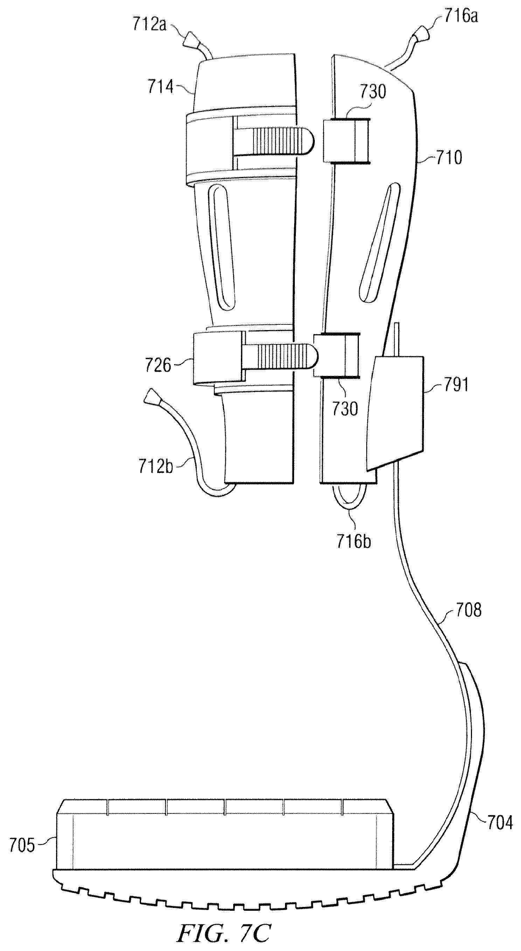

System **702** may include strut **708** positioned substantially behind a subject's leg when the leg is secured in system **702**. At least a portion of strut **708** may be fixedly connected to and/or contiguous with a posterior portion of platform **706**. Strut **708** may have an S-shaped profile as shown in Figure 7C or it may form and "L" with platform **706** (not expressly illustrated). At least a portion of strut **708** extends from a posterior portion of platform **706** in the same plane as platform **706** or a plane generally parallel to the plane of platform **706** ("horizontal" portion). At least a portion of strut **708** rises above the plane of platform **706** and generally perpendicular to the plane of platform **706** ("vertical" portion). This vertical portion may have a lengthwise axis that is approximately parallel to the lengthwise axis of a subject's leg (e.g., lower leg). Strut **708** may include bend **707** interposed between the horizontal and vertical portions of strut **708**. Bend **707** may be configured to bend or curve upward from platform **706** in a manner that permits a subject a normal or generally normal stride (e.g., heel to toe contact with the ground). Strut **708** may comprise one or more rigid materials capable of bearing a subject's weight. Examples of such materials include, without limitation, steel, a steel alloy, aluminum, an aluminum alloy, titanium, a titanium alloy, carbon fiber, wood, an aramid fiber, a para-aramid fiber (e.g., KEVLAR), fiberglass, and combinations thereof. While strut **708** may be rigid to support a subject's weight, some resilience/flexibility may be tolerated and/or desirable in some embodiments.

As depicted, cuff **710** may comprise anterior bladder **712**, anterior shell **714**, posterior bladder **716**, posterior shell **718**, and tension adjustment fastener **726**. Bladder **712** may include one or more protrusions **713**. Shell **714** may include one or more apertures **715**. Bladder **716** may include one or more protrusions **717**. Shell **718** may include one or more apertures **719**. Bladder protrusions **713** and **717** may be aligned with shell apertures **715** and **719**, respectively to reduce or prevent, for example, movement of bladders relative to shells. Bladders **712** and/or **716** may independently comprise flexible, elastomeric, and/or resilient materials including, for example, polyurethane, polyethylene, neoprene, ethylene vinyl acetate, foam, silicone, rubber, and the like. Each bladder may have an inlet **(712a** and/or **716a)** and an outlet **(712b** and/or **716b).** System **702** may be custom fit to a subject by mounting system **702** on the subject and subsequently injecting a fill material into a bladder inlet **(712a** and/or **716a).** Fill material may be injected in some embodiment until it begins to be released from bladder outlet **(712b** and/or **716b).** In some embodiments, a fill material may be flowable (*e.g.*, a liquid) when it enters a bladder. A fill material may later convert and/or be converted to a resilient, semi-resilient, or non-resilient gel or non-liquid (*e.g.*, solid) material. Examples of fill materials include, for example, foam, spray foam, gel foam, and/or combinations thereof.

Shells **714** and/or **718** may independently comprise a moldable plastic. In some embodiments, shells **714** and/or **718** may independently comprise steel, a steel alloy, aluminum, an aluminum alloy, titanium, a titanium alloy, carbon fiber, an aramid fiber, a para-aramid fiber (*e.g.*, KEVLAR), fiberglass, and combinations thereof. Shells **714** and/or **718** comprising a moldable material may be custom fit to a subject. A moldable plastic may include, for example, a thermoplastic (*e.g.*, capable of more than one cycle of melting, molding, and setting) and/or a thermosetting plastic (*e.g.*, capable of one cycle of melting, molding, and setting). Bladders **712** and/or **716** may be adhered, welded, bonded, stitched, or otherwise fixed to shells **714** and/or **718,** respectively.

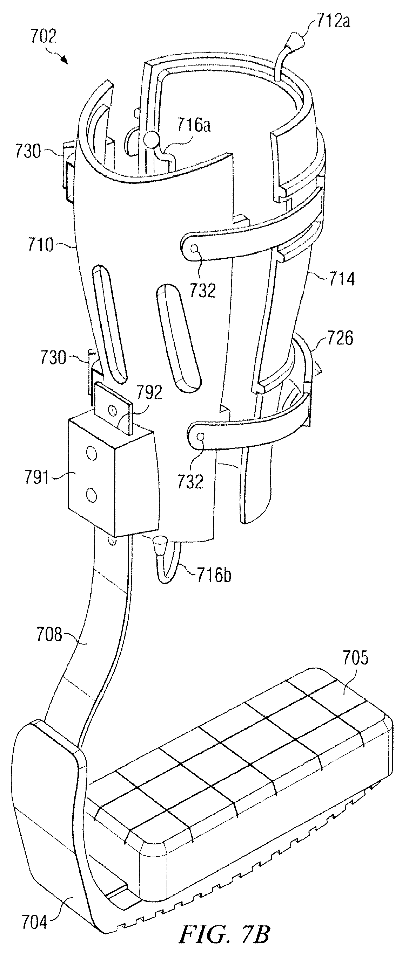

As shown in FIGURE 7B, shell **718** may be fixedly attached to support **791**. Support **791** may be attached to strut **708 -** one or more screws **724** may be secured to support **791** through one or more holes **725** in strut **708**. As shown in FIGURE 7B, a plurality of holes **725** along a vertical axis of strut **708** affords a health care worker and/or a subject the opportunity to adjust the height of cuff **710.**

Tension adjustment fastener **726** may include strap **728,** cinch **730,** and anchor **732**. Strap **728** maybe fixed to posterior shell **718** via anchor **732**. Cinch **730** may be fixed to posterior shell **718** at a point along the circumference of shell **718** and spaced away from anchor **732**. Cinch **730** may be configured to receive and releasably grip strap **728**. In use, strap **728** may be threaded through cinch **730** and pulled tight to apply a desired amount of pressure on the subject's lower leg. Cinch **730** may then be closed to fix strap **728** in its position. System **702** may include one, two (pictured), three or more tension adjustment fasteners **726.**

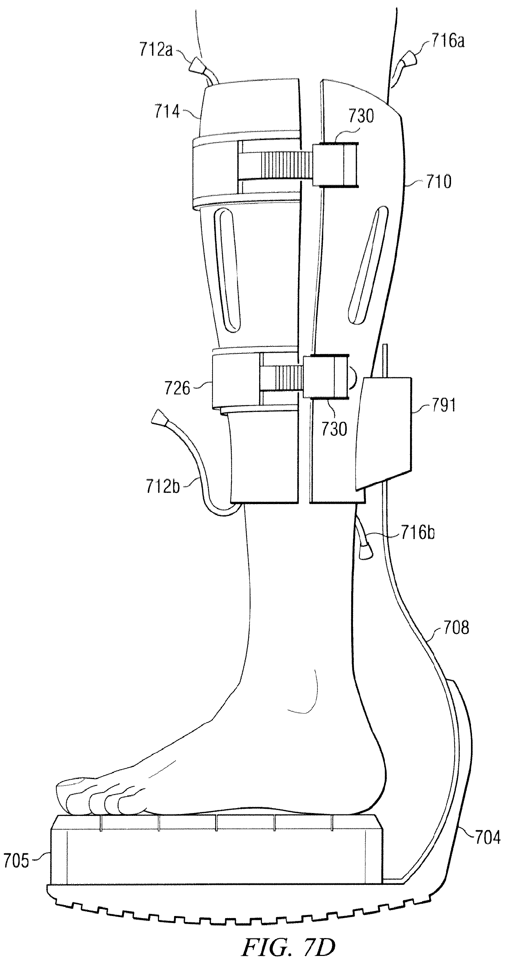

FIGURES 7D and 7E illustrate an example load redistribution system **702** for ambulation of a subject having a lower extremity impairment, in accordance with embodiments of the present disclosure. As shown in FIGURE 7D, system **702** may include pad **705** positioned to apply just enough pressure to the bottom of a subject's foot (*e.g.,* the toes and/or the ball of the foot) to hold it generally perpendicular to the long axis of the lower leg (*e.g.,* tibia and/or fibula). As shown in FIGURE 7E, one or more segments of pad **705** may be removed to accommodate the particular desires or needs of a subject with ulcer **773** or other foot injury. Pad **705** may span up to the entire gap **775**. Pad **705** may comprise flexible, elastomeric, and/or resilient materials including, for example, polyurethane, polyethylene, neoprene, ethylene vinyl acetate, foam, silicone, rubber, and the like.

FIGURES 7F and 7G illustrate exploded views of an example load redistribution system **702** for ambulation of a subject having a lower extremity impairment, in accordance with embodiments of the present disclosure. As shown in FIGURE 7G, pad shell **718** may include protrusions **733a** that complement slots **733b** in pad shell **714**. When pad shells **714** and **718** come together (*e.g.,* around a subject's leg), protrusions **733a** fit into corresponding slots **733b** and may stabilize the pad shell sections relative to each other.

FIGURE 7H illustrates an example load redistribution system **702** for ambulation of a subject having a lower extremity impairment, in accordance with embodiments of the present disclosure. As shown in FIGURE 7H, system **702** may include shell **795** configured to shield and/or protect a subject's foot from incidental contact with other objects. Shell **795** may be secured to strut **708** with straps **796** and snaps **797** as illustrated. Additional straps, snaps, and/or other fasteners may be added to secure shell **795** (*e.g.*, between shell **795** and outsole **704** and/or platform **708.**

Persons skilled in the art may make various changes in the shape, size, number, and/or arrangement of parts without departing from the scope of the instant disclosure. For example, the position and number of struts, pads, collars, and/or cuffs may be varied. In some embodiments, pads, pad shells, collars, and/or cuffs may be interchangeable. Interchangeability may allow the pressure exerted on a lateral surface of a subject's leg to be custom adjusted (e.g., by substituting larger or smaller pads). In addition, the size of a device and/or system may be scaled up (*e.g.,* to be used for adult subjects) or down (*e.g.,* to be used for juvenile subjects) to suit the needs and/or desires of a practitioner. Also, where ranges have been provided, the disclosed endpoints may be treated as exact and/or approximations as desired or demanded by the particular embodiment. In addition, it may be desirable in some embodiments to mix and match range endpoints. All or a portion of a device and/or system for redistributing a load may be configured and arranged to be disposable, serviceable, interchangeable, and/or replaceable. These equivalents and alternatives along with obvious changes and modifications are intended to be included within the scope of the present disclosure. Accordingly, the foregoing disclosure is intended to be illustrative, but not limiting, of the scope of the invention as defined by the following claims.

**Claims**

1. A system (101; 201; 301; 501; 602; 702) for redistributing weight away from a subject's foot, the system comprising:

   a platform (106; 306; 606; 706);
   a single vertical support (108, 308, 608, 708) fixed to the platform (106; 306; 606; 706) and extending upwardly from the platform (106; 306; 606; 706); and
   at least one cuff (110,310; 510; 610; 710) configured to surround and releasably grip at least a portion of a subject's leg other than the foot;
   **characterized in that** the at least one cuff (110,310; 510; 610; 710) is adjustably mounted to the single vertical support (108, 308, 608, 708) so as to be mounted at a vertical position along the single vertical support (108, 308, 608, 708) sufficient to suspend the subject's foot in a non-weight-bearing position above the platform (106; 306; 606; 706) during ambulation, wherein the platform (106; 306; 606; 706), the single vertical support (108, 308, 608, 708), and the at least one cuff (110, 310; 510; 610; 710) together are configured to bear at least the subject's full weight.

2. A system for redistributing weight away from a subject's foot according to Claim 1, wherein the single vertical support (108; 308; 608; 708) comprises a strut positioned relative to the subject's leg on which the system is worn opposite the subject's sagittal plane or opposite the subject's coronal plane, positioned generally outside the subject's leg on which the system is worn.

3. A system for redistributing weight away from a subject's foot according to Claim 1 or 2, wherein the single vertical support (108; 308; 608; 708) comprises a strut positioned generally behind the subject's leg on which the system is worn.

4. A system for redistributing weight away from a subject's foot according to Claim 3, wherein the strut has an S-curve profile.

5. A system for redistributing weight away from a subject's foot according to one of the preceding claims, wherein the at least one cuff (110; 310; 610; 710) comprises an anterior shell (114; 314; 614; 714), an opposing posterior shell (118; 314; 614; 718), and at least one tension adjustment fastener (126; 326; 626; 726) configured to releasably connect the anterior shell (114; 614; 714) and the posterior shell (118; 314; 614; 718), wherein the anterior shell (114; 314; 614; 714) and the posterior shell (118; 314; 614; 718) are configured to combine to surround the circumference of the subject's leg and wherein the anterior shell and/or the posterior shell comprise a moldable plastic selected from the group consisting of a thermoplastic, a thermosetting plastic, and combinations thereof.

6. A system for redistributing weight away from a subject's foot according to one of the preceding claims, wherein the anterior shell (114; 314; 614; 714) or the posterior shell (118; 318; 618; 718) is adjustably mounted to the single vertical support (108; 308; 608; 708).

7. A system for redistributing weight away from a subject's foot according to one of the preceding claims, wherein the at least one cuff (110; 310; 610; 710) further comprises at least one pad (112; 312; 612) fixed to the anterior shell (114; 314; 614; 714) and at least one pad (116; 316; 616) fixed to the posterior shell (118; 318; 618; 718), and wherein the pads (112; 312; 612; 116; 316; 616) independently comprise a material selected from the group consisting of polyurethane, polyethylene, neoprene, ethylene vinyl acetate, foam, silicone, rubber, and combinations thereof.

8. A system for redistributing weight away from a subject's foot according to one of the preceding claims 1-5, wherein the at least one cuff (710) further comprises at least one bladder (712) fixed to the anterior shell (714) and at least one bladder (716) fixed to the posterior shell (718).

9. A system for redistributing weight away from a subject's foot according to one of the preceding claims, further comprising an outsole (104; 304; 604; 704) fixed to the platform (106) and configured to contact the ground during ambulation.

10. A system for redistributing weight away from a subject's foot according to one of the preceding claims, further comprising a foot pad (705) fixed to the platform (704) and configured to contact at least a portion of the subject's foot and to hold it approximately perpendicular to the longitudinal axis of the subject's lower leg.

**11.** A system for redistributing weight away from a subject's foot according to one of the preceding claims, further comprising a second cuff (140), wherein the second cuff (140) is configured to surround and releasably grip at least a second portion of a subject's leg other than the foot and to be mounted to the vertical support (108, 138) at a vertical position along the single vertical support (108, 138) sufficient to suspend a subject's foot in a non-weight bearing position above the platform (106) during ambulation.

**12.** A system for redistributing weight away from a subject's foot according to one of the preceding claims; wherein the single vertical support comprises:

a first strut (108);
a hinge (136) attached to the upper end of the first strut (108);
a second strut (138) rotatably attached to the hinge (136); and
a second cuff (140), wherein the second cuff is configured to surround and releasably grip at least a second portion of a subject's leg other than the foot and to be mounted to the second strut at a vertical position along the second strut sufficient to cooperate with the first cuff (110; 610; 710) to suspend a subject's foot in a non-weight-bearing position above the platform (106) during ambulation.

**13.** A system for redistributing weight away from a subject's foot according to one of the preceding claims, further comprising a mated shoe comprising a lift configured to vertically align the suspended foot and the other foot.

**14.** A method for facilitating ambulation of a subject having a leg with an impaired lower extremity, the method comprising:

suspending at least a portion of the impaired lower extremity in a non-weight bearing position using a load redistribution system (101; 201; 301; 501; 602; 702) according to one of the preceding claims; and
redistributing the weight to one or more unimpaired regions of the leg using the load redistribution system.

**Patentansprüche**

**1.** System (101; 201; 301; 501; 602; 702) zur Umverteilung von Gewicht, um den Fuß eines Patienten zu entlasten, wobei das System umfasst:

eine Plattform (106; 306; 606; 706);
einen einzelnen vertikalen Träger (108, 308, 608, 708), der an der Plattform (106; 306; 606; 706) fixiert ist und sich von der Plattform (106; 306; 606; 706) nach oben erstreckt; und
mindestens eine Manschette (110, 310; 510; 610; 710), die dafür ausgelegt ist, zumindest einen Abschnitt eines Beins eines Patienten, bei dem es sich nicht um den Fuß handelt, zu umschließen und lösbar zu greifen; **dadurch gekennzeichnet, dass** die mindestens eine Manschette (110, 310; 510; 610; 710) solchermaßen justierbar an dem einzelnen vertikalen Träger (108, 308, 608, 708) montiert ist, dass sie in einer vertikalen Position entlang des einzelnen vertikalen Trägers (108, 308, 608, 708) montiert werden kann, die ausreicht, um den Fuß eines Patienten während des Gehens in einer Position über der Plattform (106; 306; 606; 706) in der Schwebe zu halten, wo er kein Gewicht zu tragen hat; wobei die Plattform (106; 306; 606; 706), der einzelne vertikale Träger (108, 308, 608, 708) und die mindestens eine Manschette (110, 310; 510; 610; 710) dafür ausgelegt sind, zusammen zumindest das volle Gewicht des Patienten zu tragen.

**2.** System nach Anspruch 1, zur Umverteilung von Gewicht, um den Fuß eines Patienten zu entlasten, wobei der einzelne vertikale Träger (108; 308; 608; 708) eine Strebe umfasst, die in Bezug auf das Bein des Patienten, an dem das System angelegt wird, gegenüber der Sagittalebene oder gegenüber der Koronalebene des Patienten positioniert ist, allgemein außerhalb des Beins des Patienten positioniert, an dem das System angelegt wird.

**3.** System nach Anspruch 1 oder 2, zur Umverteilung von Gewicht, um den Fuß eines Patienten zu entlasten, wobei der einzelne vertikale Träger (108; 308; 608; 708) eine Strebe umfasst, die allgemein hinter dem Bein positioniert ist, an dem das System angelegt wird.

**4.** System nach Anspruch 3, zur Umverteilung von Gewicht, um den Fuß eines Patienten zu entlasten, wobei die Strebe ein S-Kurvenprofil aufweist.

**5.** System nach einem der vorangehenden Ansprüche, zur Umverteilung von Gewicht, um den Fuß eines Patienten zu entlasten,
wobei die mindestens eine Manschette (110; 310; 610; 710) eine vordere Schale (114; 314; 614; 714), eine hintere Schale (118; 314; 614; 718) und mindestens einen Spannungsjustierverschluss (126; 326; 626; 726) umfasst, der dafür ausgelegt ist, die vordere Schale (114; 614; 714) und die hintere Schale (118; 314; 614; 718) lösbar zu verbinden,
wobei die vordere Schale (114; 314; 614; 714) und die hintere Schale (118; 314; 614; 718) dafür ausgelegt sind, den Umfang des Beins des Patienten gemeinsam zu umschließen, und
wobei die vordere Schale und/oder die hintere Schale einen formbaren Kunststoff umfassen, der ausgewählt ist aus der Gruppe bestehend aus einem thermoplastischen Material, einem duroplastischen Material und Kombinationen davon.

6. System nach einem der vorangehenden Ansprüche, zur Umverteilung von Gewicht, um den Fuß eines Patienten zu entlasten, wobei die vordere Schale (114; 314; 614; 714) oder die hintere Schale (118; 318; 618; 718) justierbar an dem einzelnen vertikalen Träger (108; 308; 608; 708) montierbar ist.

7. System nach einem der vorangehenden Ansprüche, zur Umverteilung von Gewicht, um den Fuß eines Patienten zu entlasten, wobei die mindestens eine Manschette (110; 310; 610; 710) ferner mindestens ein Polster (112; 312; 612), das an der vorderen Schale (114; 314; 614; 714) fixiert ist, und mindestens ein Polster (116; 316), das an der hinteren Schale (118; 318; 618; 718) fixiert ist, aufweist, und wobei die Polster (112; 312; 612; 116; 316; 616) jeweils unabhängig ein Material umfassen, das ausgewählt ist aus der Gruppe bestehend aus Polyurethan, Polyethylen, Neopren, Ethylenvinylacetat, Schaumstoff, Silikon, Gummi und Kombinationen davon.

8. System nach einem der vorangehenden Ansprüche 1-5, zur Umverteilung von Gewicht, um den Fuß eines Patienten zu entlasten, wobei die mindestens eine Manschette (710) ferner mindestens ein Kissen (712), das an der vorderen Schale (714) fixiert ist, und mindestens ein Kissen (716), das an der hinteren Schale (718) fixiert ist, umfasst.

9. System nach einem der vorangehenden Ansprüche, zur Umverteilung von Gewicht, um den Fuß eines Patienten zu entlasten, ferner eine Laufsohle (104; 304; 604; 704) umfassend, die an der Plattform (106) fixiert ist und dafür ausgelegt ist, während des Gehens den Boden zu berühren.

10. System nach einem der vorangehenden Ansprüche, zur Umverteilung von Gewicht, um den Fuß eines Patienten zu entlasten, ferner eine Fußauflage (705) umfassend, die an der Plattform (704) fixiert ist und dafür ausgelegt ist, zumindest einen Abschnitt des Fußes des Patienten zu berühren und diesen ungefähr senkrecht zur Längsachse des Unterschenkels des Patienten zu halten.

11. System nach einem der vorangehenden Ansprüche, zur Umverteilung von Gewicht, um den Fuß eines Patienten zu entlasten, ferner eine zweite Manschette (140) umfassend, wobei die zweite Manschette (140) dafür ausgelegt ist, zumindest einen zweiten Abschnitt eines Beins eines Patienten, bei dem es sich nicht um den Fuß handelt, zu umschließen und lösbar zu greifen und in einer vertikalen Position entlang des einzelnen vertikalen Trägers (108, 138) an dem vertikalen Träger (108, 138) montiert zu werden, die ausreicht, um den Fuß eines Patienten während des Gehens in einer Position über der Plattform (106) in der Schwebe zu halten, wo er kein Gewicht zu tragen hat.

12. System nach einem der vorangehenden Ansprüche, zur Umverteilung von Gewicht, um den Fuß eines Patienten zu entlasten; wobei der einzelne vertikale Träger umfasst:

eine erste Strebe (108);
ein Gelenk (136), das am oberen Ende der ersten Strebe (108) befestigt ist;
eine zweite Strebe (138), die drehbar an dem Gelenk (136) angebracht ist; und
eine zweite Manschette (140), wobei die zweite Manschette dafür ausgelegt ist, zumindest einen zweiten Abschnitt eines Beins eines Patienten, bei dem es sich nicht um den Fuß handelt, zu umschließen und lösbar zu greifen und in einer vertikalen Position entlang der zweiten Strebe an der zweiten Strebe montiert zu werden, die ausreicht, um mit der ersten Manschette (110; 610; 710) zusammenzuwirken, um einen Fuß des Patienten während des Gehens in einer Position über der Plattform (106) in der Schwebe zu halten, wo er kein Gewicht zu tragen hat.

13. System nach einem der vorangehenden Ansprüche, zur Umverteilung von Gewicht, um den Fuß eines Patienten zu entlasten, ferner einen dazu passenden Schuh umfassend, der eine Erhöhung umfasst, die dafür ausgelegt ist, den in der Schwebe gehaltenen Fuß und den anderen Fuß vertikal aufeinander abzustimmen.

**14.** Verfahren, um einem Patienten mit einem Bein, das eine geschädigte untere Gliedmaße aufweist, das Gehen zu erleichtern, wobei das Verfahren umfasst:

schwebend Halten zumindest eines Abschnitts der geschädigten unteren Gliedmaße in einer Position, wo sie kein Gewicht zu tragen hat, unter Verwendung eines Lastumverteilungssystems (101; 201; 301; 501; 602; 702) nach einem der vorangehenden Ansprüche; und
Umverteilen des Gewichts auf eine oder mehrere ungeschädigte Regionen des Beins unter Verwendung des Lastumverteilungssystems.

**Revendications**

**1.** Un système (101 ; 201 ; 301 ; 501 ; 602 ; 702) destiné à la redistribution du poids autour du pied d'un sujet, le système comprenant :

une plateforme (106 ; 306 ; 606 ; 706) ;
un support vertical unique (108, 308, 608, 708) fixé à la plateforme (106 ; 306 ; 606 ; 706) et s'étendant vers le haut à partir de la plateforme (106 ; 306 ; 606 ; 706) ; et
au moins un manchon (110, 310 ; 510 ; 610 ; 710) conçu de façon à entourer et à maintenir de façon libérable au moins une partie d'une jambe d'un sujet autre que le pied ;
**caractérisé en ce que** l'au moins un manchon (110, 310 ; 510 ; 610 ; 710) est monté de façon réglable sur le support vertical unique (108, 308, 608, 708) de façon à être monté dans une position verticale le long de l'au moins un support vertical unique (108, 308, 608, 708) suffisant pour suspendre le pied d'un sujet dans une position non portante au-dessus de la plateforme (106 ; 306 ; 606 ; 706) durant l'ambulation, dans lequel la plateforme (106 ; 306 ; 606 ; 706), le support vertical unique (108, 308, 608, 708) et l'au moins un manchon (110, 310 ; 510 ; 610 ; 710) sont conçus ensemble pour porter au moins le poids total du sujet.

**2.** Un système destiné à la redistribution du poids autour du pied d'un sujet selon la revendication 1, dans lequel le support vertical unique (108 ; 308 ; 608 ; 708) comprend une jambe de force positionnée par rapport à la jambe du sujet sur laquelle le système est porté en regard du plan sagittal du sujet ou en regard du plan coronal du sujet, positionné généralement à l'extérieur de la jambe du sujet sur laquelle le système est porté.

**3.** Un système destiné à la redistribution du poids autour du pied d'un sujet selon la revendication 1 ou 2, dans lequel le support vertical unique (108 ; 308 ; 608 ; 708) comprend une jambe de force positionnée généralement derrière la jambe sur laquelle le système est porté.

**4.** Un système destiné à la redistribution du poids autour du pied d'u sujet selon la revendication 3, dans lequel la jambe de force présente un profil à courbe en S.

**5.** Un système destiné à la redistribution du poids autour du pied d'un sujet selon une des revendications précédentes, dans lequel l'au moins un manchon (110 ; 310 ; 610 ; 710) comprend une coque antérieure (114 ; 314 ; 614 ; 714), une coque postérieure opposée (118 ; 314 ; 614 ; 718) et au moins un élément de fixation de réglage de la tension (126 ; 326 ; 626 ; 726) conçu pour raccorder la coque antérieure de façon libérable (114 ; 614 ; 714) et la coque postérieure (118 ; 314; 614 ; 718),
dans lequel la coque antérieure (114 ; 314 ; 614 ; 714) et la coque postérieure (118 ; 314 ; 614 ; 718) sont conçues pour se combiner pour entourer la circonférence de la jambe du sujet et
dans lequel la coque antérieure et/ou la coque postérieure comprennent un plastique moulable choisi dans le groupe composé d'un thermoplastique, d'un plastique thermodurcissable et de leurs combinaisons.

**6.** Un système destiné à la redistribution du poids autour du pied d'un sujet selon une des revendications précédentes, dans lequel la coque antérieure (114 ; 314 ; 614 ; 714) ou la coque postérieure (118 ; 318 ; 618 ; 718) est montée de façon réglable sur le support vertical unique (108 ; 308 ; 608 ; 708).

**7.** Un système destiné à la redistribution du poids autour du pied d'un sujet selon une des revendications précédentes, dans lequel l'au moins un manchon (110 ; 310 ; 610 ; 710) comprend en outre au moins un coussin (112; 312; 612) fixé à la coque antérieure (114 ; 314 ; 614 ; 714) et au moins un coussin (116; 316; 616) fixé à la coque postérieure (118 ; 318 ; 618 ; 718), et dans lequel les coussins (112 ; 312 ; 612 ; 116 ; 316 ; 616) comprennent de façon indépendante un matériau sélectionné dans le groupe composé de polyuréthane, polyéthylène, néoprène, acétate de vinyle-éthylène, mousse, silicone, caoutchouc et leurs combinaisons.

8.  Un système destiné à la redistribution du poids autour du pied d'un sujet selon une des revendications précédentes 1 à 5, dans lequel l'au moins un manchon (710) comprend en outre au moins une vessie (712) fixée à la coque antérieure (714) et au moins une vessie (716) fixée à la coque postérieure (718).

9.  Un système destiné à la redistribution du poids autour du pied d'un sujet selon une des revendications précédentes, comprenant en outre une semelle extérieure (104 ; 304 ; 604 ; 704) fixée à la plateforme (106) et conçue pour entrer en contact avec le sol durant l'ambulation.

10. Un système destiné à la redistribution du poids autour du pied d'un sujet selon une des revendications précédentes, comprenant en outre un coussin de pied (705) fixé à la plateforme fixe (704) et conçu pour entrer en contact avec au moins une partie du pied du sujet et pour le maintenir approximativement perpendiculaire à l'axe longitudinal de la partie inférieure de la jambe du sujet.

11. Un système destiné à la redistribution du poids autour du pied d'un sujet selon une des revendications précédentes, comprenant en outre un second manchon (140), dans lequel le second manchon (140) est conçu pour entourer et maintenir de façon libérable au moins une seconde partie de la jambe d'un sujet autre que le pied et pour être monté sur le support vertical (108, 138) dans une position verticale le long du support vertical unique (108, 138) suffisante pour suspendre un pied d'un sujet dans une position non portante au-dessus de la plateforme (106) durant l'ambulation.

12. Un système destiné à la redistribution du poids autour du pied d'un sujet selon une des revendications précédentes, dans lequel le support vertical unique comprend :

    une première jambe de force (108) ;
    une articulation (136) fixée à l'extrémité supérieure de la première jambe de force (108);
    une seconde jambe de force (138) fixée en rotation à l'articulation (136) ; et
    un second manchon (140), dans lequel le second manchon est conçu pour entourer et maintenir de façon libérable au moins une seconde partie de la jambe d'un sujet autre que le pied et pour être monté à la seconde jambe de force dans une position verticale le long de la seconde jambe de force suffisante pour coopérer avec le premier manchon (110 ; 610 ; 710) pour suspendre un pied d'un sujet dans une position non portante au-dessus de la plateforme (106) durant l'ambulation.

13. Un système destiné à la redistribution du poids autour du pied d'un sujet selon une des revendications précédentes, comprenant en outre une chaussure appariée comprenant un dispositif de levage conçu pour aligner verticalement le pied suspendu et l'autre pied.

14. Un procédé destiné à faciliter l'ambulation d'un sujet présentant une jambe comportant une extrémité inférieure handicapée, le procédé comprenant :

    la suspension d'au moins une partie de l'extrémité inférieure handicapée dans une position non portante à l'aide d'un système de redistribution de la charge (101 ; 201 ; 301 ; 501 ; 602 ; 702) selon une des revendications précédentes ; et
    la redistribution du poids sur une ou plusieurs régions non handicapées de la jambe à l'aide du système de redistribution de la charge.

*1/25*

*FIG. 1A*

*FIG. 1B*

146    142

148    144

116    112

118    114

*FIG. 1C*

FIG. 1D

*FIG. 2A*

*FIG. 2B*

FIG. 3A

FIG. 3B

FIG. 3C

FIG. 3D

*FIG. 4*

FIG. 5A

FIG. 5B

602

628

633

626

618

690

633

614

608

606

604

*FIG. 6A*

*FIG. 6B*

FIG. 6C

FIG. 6D

*FIG. 6E*

619
617
613
615
618
614
608
690
624
636
624

*FIG. 6F*

630
626
618
614
630
608
690
*FIG. 6G*
624

*FIG. 6H*

712a — 716a
710
730
714
726
730
791
712b
716b
708
707
705
704

*FIG. 7A*

702

712a

716a

730

710

732

714

730

792

791

726

732

716b

708

705

704

*FIG. 7B*

*FIG. 7C*

*FIG. 7D*

*FIG. 7E*

FIG. 7F

FIG. 7G

FIG. 7H

**REFERENCES CITED IN THE DESCRIPTION**

**Patent documents cited in the description**

- US 20050054962 A
- US 6997891 B
- FR 2432306

- US 20030216675 A
- WO 9749359 A
- US 20080269656 A